# FASCICULE DE BREVET EUROPEEN

(11) **EP 3 861 039 B1**
(45) Date de publication et mention de la délivrance du brevet: **01.11.2023**
(21) Numéro de dépôt: 19795285.6
(22) Date de dépôt: 30.09.2019
(51) Int. Cl.: C08F 290/06, C08F 220/20, G02B 1/04, A61L 27/18, A61L 27/52

(54) **COMPOSITIONS D'HYDROGELS**
HYDROGELZUSAMMENSETZUNGEN
HYDROGEL COMPOSITIONS

(30) Priorité: 02.10.2018 FR 1859125
(43) Date de publication de la demande: 11.08.2021
(73) Titulaire: Ophtalmic Compagnie, 93420 Villepinte (FR); Université de Haute Alsace, 68200 Mulhouse (FR)
(72) Inventeur: STUMBÉ, Jean-François, 67200 STRASBOURG (FR); JAGU, Romain, 44530 DREFFÉAC (FR); RUMIGNY, Jean-François, 56450 SURZUR (FR); BISTAC, Sophie, 68990 GALFINGUE (FR); COUMES, Fanny, 11250 LEUC (FR)
(74) Mandataire: Novagraaf Technologies
(86) Numéro de dépôt international: PCT/FR2019/052303
(87) Numéro de publication internationale: WO 2020/070423

(56) Documents cités:
- JP-A- 2015 044 955
- HAO YING ET AL: "Hyperbranched polyglycerol/poly(acrylic acid) hydrogel for the efficient removal of methyl violet from aqueous solutions", JOURNAL OF APPLIED POLYMER SCIENCE, vol. 133, no. 5, 12 octobre 2015 (2015-10-12), pages n/a-n/a, XP055592967, US ISSN: 0021-8995, DOI: 10.1002/app.42951
- OUDSHOORN MARION H M ET AL: "Preparation and characterization of structured hydrogel microparticles based on cross-linked hyperbranched polyglycerol", LANGMUIR, AMERICAN CHEMICAL SOCIETY, US, vol. 23, no. 23, 6 novembre 2007 (2007-11-06), pages 11819-11825, XP002543499, ISSN: 0743-7463, DOI: 10.1021/LA701910D [extrait le 2007-10-10]

## Description

### Domaine technique

La présente invention se rapporte à des compositions réticulables et réticulées, ainsi qu'à un hydrogel susceptible d'être obtenu à partir de la composition réticulée.

La présente invention trouve des applications industrielles dans le domaine des matériaux biocompatibles, et notamment celui des lentilles oculaires.

Dans la description ci-dessous, les références entre crochets (**[ ])** renvoient à la liste des références présentées à la fin du texte.

### Etat de la technique

Une lentille de contact, portée sur l'œil, sert à corriger des défauts de vision tels que la myopie, l'astigmatisme, ou l'hypermétropie. Cependant, l'œil humain a besoin de conserver un certain taux d'hydratation et de circulation d'oxygène. Ainsi la lentille en contact avec l'œil doit répondre à un cahier des charges incluant mais ne se limitant pas à une bonne perméabilité à l'oxygène, un bon confort, une bonne rétention d'eau ou encore un caractère hydrophile.

Les lentilles de contact peuvent être classées en deux catégories : les lentilles de contact rigides, et les lentilles de contact souples, comme les lentilles hydrogel ou les lentilles en hydrogel silicone.

Un hydrogel est un système polymère réticulé hydraté qui contient de l'eau dans un état d'équilibre. Il est typiquement perméable à l'oxygène et biocompatible, ce qui en fait un matériau préféré pour produire des dispositifs biomédicaux et en particulier des lentilles de contact ou intraoculaires. Les lentilles souples en hydrogel sont généralement fabriquées à partir d'un nombre limité de monomères de base. Leur choix va dépendre du caractère final que souhaite privilégier le fabricant de lentille :
- meilleure perméabilité à l'oxygène augmentant avec la teneur en eau ou avec la teneur en silicone,
- meilleure résistance aux dépôts lipidiques et protéiques notamment,
- meilleure résistance à la déshydratation, qui a tendance à augmenter avec la teneur en eau.

En général, lors de la production de lentilles de contact polymères, une composition polymérisable de précurseurs de lentille est polymérisée pour former un produit de lentille de contact, qui est ensuite traité pour former une lentille de contact hydratée. Par exemple, la lentille peut être préparée selon un procédé de moulage qui consiste à placer la composition de précurseur polymérisable dans un moule de la forme désirée, et peut y être polymérisée pour former une lentille de contact. La polymérisation peut être réalisée par exposition de la résine polymérisable constituée de précurseurs polymérisables à une lumière ultraviolette ou à la chaleur. Après polymérisation de la composition de précurseurs de lentille, les sections de moule sont séparées, et la lentille de contact polymérisée peut être démoulée, c'est-à-dire enlevée ou délissée de la section de moule.

Souvent, les lentilles de contact hydrogel classiques sont le produit polymérisé d'une composition de précurseurs de lentille contenant des monomères hydrophiles tels que le méthacrylate de 2-hydroxyéthyle (HEMA), l'acide méthacrylique (MAA), et la N-vinylpyrrolidone (NVP), pouvant contenir en outre des additifs non réactifs afin d'améliorer certaines propriétés telles que la rétention d'eau et les propriétés mécaniques, comme l'acool polyvinylique ou poly(alcool cinylique) (PVA), la polyvinylpyrrolidone (PVP) et le poly(éthylène glycol) (PEG), et leurs combinaisons. Les compositions de précurseurs contiennent également fréquemment un ou plusieurs catalyseurs et agents de réticulation.

Plus récemment, une nouvelle génération de polymères a été développée pour augmenter encore la perméabilité à l'oxygène. Ces matériaux sont basés sur la copolymérisation de monomères (méth)acrylates ou di(méth)acrylates fonctionnalisés par des groupements silicones avec des comonomères hydrophiles tels que l'HEMA. Les lentilles produites à partir de ces matériaux ont été initialement conçues pour un usage prolongé, avec un port continu sur 24 heures durant 15 à 30 jours. Bien qu'ayant réussi à augmenter la perméabilité à l'oxygène, ces nouveaux matériaux souffrent encore de limitations telles que l'adhésion ou le dépôt lipidique et protéique et la sécheresse, ce qui diminue le confort des yeux. XP055592967 décrit un hydrogel obtenu par polymérisation d'une composition réticulable en présence d'eau. Ladite composition comprend de l'acide acrylique, un polyol de type polyglycérol hyperramifié comprenant en moyenne plus de 1 groupement hydroxyle fonctionnalisé avec un groupement portant une fonction C=C polymérisable de type méthacrylate issue du méthacrylate de glycidyl et un amorceur radicalaire de type rédox pouvant amorcer la polymérisation par voie rédox.

Il existe donc un réel besoin de nouveaux matériaux palliant les défauts, inconvénients et obstacles de l'art antérieur, en particulier présentant des propriétés particulièrement intéressantes en terme de perméabilité à l'oxygène, teneur en eau, perte en eau, module d'Young, perméabilité aux ions, résistance en traction, élongation à la rupture, coefficient de friction et rugosité de surface.

### Description de l'invention

Au terme d'importantes recherches, le Demandeur est parvenu à mettre au point un nouveau matériau répondant aux besoins précités, et de ce fait particulièrement adapté pour une utilisation pour la fabrication de lentilles de contact.

De manière surprenante, le Demandeur a réussi à créer un hydrogel, transparent, capable de gonfler en milieu aqueux, ayant une bonne résistance au dessèchement, et montrant des propriétés très intéressantes par rapport aux matériaux existants, notamment en utilisant de nouveaux polymères fonctionnalisés consistant en des oligoglycérols ou des polyglycérols, notamment des polyglycérols linéaires, ramifiés ou hyperramifiés ou des mélanges de polyglycérol hyperramifié avec des oligoglycérols ou avec des polyglycérols linéaires, en tant que.constituant polymère principal ou comme constituant secondaire.

Ces nouveaux polymères présentent l'avantage, du fait de leur forte fonctionnalisation, de présenter une multitude de propriétés, leur permettant par exemple d'être utilisés comme un agent de réticulation, et/ou comme une espèce hydrophile et d'être fortement perméable à l'oxygène à l'état hydraté.

Une multitude de combinaisons est envisageable afin d'utiliser le polymère fonctionnalisé avec un très grand nombre de molécules permettant d'obtenir un matériau hydrogel composé d'un très grand choix de fonctions agissant sur les propriétés physico-chimiques et biologiques de l'hydrogel.

Avantageusement, le dispositif médical est biocompatible, et notamment adapté à un contact avec les yeux, et hydrophile. Il présente en outre des propriétés particulièrement intéressantes en termes de perméabilité à l'oxygène, teneur en eau, perte en eau, module d'Young, perméabilité aux ions, résistance en traction, élongation à la rupture, coefficient de friction et rugosité de surface.

Ainsi, un premier objet de l'invention se rapporte à une composition réticulable comprenant, ou consistant en :
A) au moins un monomère polymérisable ou un mélange de monomères polymérisables ou un mélange de monomères et de macromonomères polymérisables portant au moins une fonction C=C polymérisable de type acrylate, méthacrylate, maléate, fumarate, itaconate, citraconate, styrénique ou vinylique ;
B) au moins un polyol choisi parmi les oligoglycérols, les polyglycérols linéaires, ramifiés et hyperramifiés, un mélange de polyglycérol hyperramifié et d'un polyol choisi parmi les oligoglycérols et les polyglycérols linéaires, ramifiés et hyperramifiés, comprenant en moyenne plus de 1 groupement hydroxyle fonctionnalisé avec un groupement portant une fonction C=C polymérisable de type isocyanatoalkyl(méth)acrylate, maléate, fumarate, itaconate, citraconate, styrénique ou vinylique ;
C) un amorceur radicalaire ou un mélange d'amorceurs radicalaires pouvant amorcer la polymérisation par voies thermiques, rédox ou photochimiques; et
D) éventuellement au moins un additif choisi parmi les antioxydants, les agents d'ajustement des propriétés physico-chimiques telles que le module d'Young, l'élongation et la contrainte à la rupture, les agents de promotion, de modulation et / ou de contrôle de la perméabilité à l'oxygène, les plastifiants, les agents humectants, comme par exemple la PVP, le PVA et le PEG, les lubrifiants, comme par exemple l'acide hyaluronique, les agents de modification de la viscosité, les agents compatibilisants, les agents de coloration, les agents filtrants, les agents anti-microbiens, les agents thérapeutiques et les anti-biofilms bactériens.

Concernant le constituant A), on entend par « monomère polymérisable », au sens de la présente invention, tout monomère susceptible de contenir au moins une fonction terminale réactive polymérisable. Il peut s'agir d'un unique type de monomère, ou d'un mélange de différents monomères, par exemple 2 types de monomères différents, ou 3 types de monomères différents, ou 4 types de monomère différents, ou plus de 4 types de monomères différents.

On entend par « macromonomères polymérisables », au sens de la présente invention, tous les oligomères possédant une fonction terminale réactive polymérisable, aussi connus sous le terme de macromonomères monotéléchéliques, ou deux fonctions terminales réactives polymérisables, également connus sous le terme oligomère α,ω-ditéléchélique, ou plus de deux fonctions polymérisables, généralement trois ou quatre.

Dans le cas d'un mélange de monomères et de macromonomères, il peut s'agir d'un mélange comprenant au moins un type de monomère et au moins un type de macromonomère. Il peut par exemple y avoir, au sein du mélange, 1 ou 2 ou 3 ou 4 ou plus de 4 type(s) de monomère(s), et 1 ou 2 ou 3 ou 4 ou plus de 4 type(s) de macromonomère(s). Il peut par exemple s'agir d'un mélange comprenant le monomère HEMA (A), réagissant avec du polyglycérol hyperramifié fonctionnalisé (B).

Le ratio entre le monomère et le macromonomère peut être adapté par l'homme du métier en fonction des propriétés souhaitées du matériau hydrogel à préparer.

Dans le constituant A), le groupe insaturé éthylénique représentant la fonction C=C polymérisable peut être choisi parmi les fonctions acrylate, méthacrylate, maléate, fumarate, itaconate, citraconate, acrylamide ou vinyle.

Le constituant A) peut notamment être hétérobifonctionnel. Dans ce cas, il peut s'agir par exemple d'un composé présentant 1 groupe acrylate et un groupe méthacrylate, ou de toute autre combinaison des groupes mentionnés ci-avant.

Dans le constituant A), le monomère ou macromonomère peut contenir un ou plusieurs, par exemple 2 ou plus de 2, groupements fluorés, silanes et/ou siloxanes.

Le monomère ou macromonomère peut être choisi par exemple parmi :
- l'acide acrylique ou l'acide méthacrylique ainsi que les alkyl (méth)acrylates et leurs dérivés, tels que le 2-hydroxyéthylméthacrylate (HEMA), le 2-hydroxyéthylacrylate (HEA), le méthylméthacrylate (MMA), le méthacrylamide, le N,N-diméthyl-diacétoneacrylamide, le 2-phosphatoéthylméthacrylate, les mono-, di-, tri-, tétra-, penta-polyéthylèneglycol acrylates ou méthacrylates, la N-(3-méthacrylamidopropyl)-N,N-diméthylamine, la N-(3-méthacrylamidopropyl)-N,N,N-triméthylamine, la N-(3-acrylamido-3-méthylbutyl)-N,N-diméthylamine, le N-méthylacrylamide, le 3-hydroxypropyl méthacrylate, le propyl méthacrylate, le propyl acrylate, le butyl méthacrylate, le butyl acrylate, le pentyl acrylate, le pentyl méthacrylate, le N-(1,1-diméthyl-3-oxobutyl)acrylamide, le 2-éthyl-2-(hydroxy-méthyl)-1,3-propanediol le triméthacrylate, le butyl(méth)acrylate, le 2-hydroxybutyl méthacrylate, le 3-hydroxy-2-naphthyl méthacrylate, le N-(formylméthyl)acrylamide, le 2-éthoxyéthyl méthacrylate, le 4-t-butyl-2-hydroxycyclohexyl méthacrylate, ou les isocyanatoalkyl(méth)acrylates comme le 2-isocyanatoéthylméthacrylate, 2-Isocyanatoéthyl Acrylate, qui sont disponibles commercialement, ou peuvent aussi être synthétisés selon la méthodologie décrite dans le document US20010005738 **([1])**, ou encore un composé choisi parmi le 4 - [(a) -phényl diazényl] phényl-2-méthacrylate (4 - [(E) -phényldiazényl] phényl-2-méthacrylate) ou le 4 - [(a) -phényl diazényl] phényl-2-méthacrylate (4 - [(E) -phényldiazényl] phényl-2-méthacrylate), qui sont décrits dans le document WO2017150786 **([2])**, ces derniers composés pouvant être présents jusqu'à environ 5% molaire des acrylates ou du mélange d'acrylates,
- les monomères maléates et fumarates, tels que l'acide maléique, l'anhydride maléique, le diméthylmaléate, le diéthylmaléate, ou les esters de maléate supérieurs dans lesquels la chaîne alkyle contient 3 à 20 atomes de carbone ainsi que l'acide fumarique, le diméthylfumarate, le diéthylfumarate ou les esters de fumarate supérieurs dans lesquels la chaîne alkyle contient 3 à 20 atomes de carbone,

- les monomères itaconates ; tels que l'acide itaconique, l'anhydride itaconique, le diméthylitaconate, ou les esters d'itaconate supérieurs dans lesquels la chaîne alkyle contient 3 à 20 atomes de carbone,
- les monomères citraconates ; tels que l'acide citraconique, l'anhydride citraconique, le diméthylcitraconate, ou les esters de citraconate supérieurs dans lesquels la chaîne alkyle contient est un groupement alkyle contenant 3 à 20 atomes de carbone,
- les monomères (méth)acryliques pouvant contenir des fonctions silicones ou silanes ; par exemple le méthacrylate de tris(triméthylsiloxy)silylpropyle, le méthacrylate de bis(triméthylsiloxy)méthylsilylpropyle, le méthacrylate de pentaméthyldisiloxanepropyle, le méthacrylate de tris(triméthylsiloxy) silylpropyloxyéthyle, le méthylacryloxyéthylcarbamate de tris(triméthylsiloxy)-silylpropyle, le méthacrylate de tris(triméthylsiloxy)silylpropylglycérol, l'acrylate de phényltétraméthyle-disiloxanyléthyle, le 3-méthacryloxypropylbis(triméthylsiloxy)méthylsilane, le méthyl-di(triméthylsiloxy)méthacryloxyméthyle silane, le (3-méthacryloxy-2-hydroxypropyloxy) propyle bis (triméthylsiloXy)méthylsilane, le 3-Acryloxypropyl Tris(Triméthylsiloxy)Silane, le 3-Acryloxypropyltrichlorosilane, 3-Méthacryloxypropyl Diméthyl Ethoxysilane, 3-Méthacryloxypropyl Méthyl Diéthoxysilane, le méthacryloxypropylbis(triméthylsiloxy)méthylsilane, le tristriméthylsilyloxysilylpropyl méthacrylate, le bis(méthacryloxypropyle) tétraméthyldisiloxane, l'acryloxyméthyltriméthylsilane, le p-(t-butyldiméthylsiloxy)styrène, le 1,3-bis(3-méthacryloxypropyl)tétrakis(triméthylsiloxy)disiloxane, le 3 triméthylsilylpropargylméthacrylate, l'acrylate de 3-(triméthoxysilyl)propyle,
- les monomères vinyliques pouvant contenir des fonctions silicones ou silanes ; par exemple le vinyl(chlorométhyl)diméthoxysilane, le vinyléthoxysiloxane-propyléthoxysiloxane, le vinyltris(1-méthoxy-2-propoxy)silane, le vinyltris(2-méthoxyéthoxy)silane, le vinyltriméthoxysilane, le vinyltriisopropoxysilane, le vinyltriéthoxysilane, le vinyltrichlorosilane, le vinyl tri-t-butoxysilane, le o-(vinyloxybutyl)-n-triéthoxysilylpropyl carbamate, le vinylméthyldiméthoxysilane, le vinylméthyldiéthoxysilane, le vinyldiméthyléthoxysilane, le 3-[tris (triméthylsiloxy)silyle] propyl vinyl carbamate, l'acide triéthoxysilylpropyl maléique ou le 1,3,5-trivinyl-1,1,3,5,5-pentaméthyltrisiloxane,
- les monomères fluorés acryliques et méthacryliques, tels que le dihydroperfluorooctyle acrylate, le 1,1-dihydroperfluorobutyle acrylate, les trihydroperfluoroalkyle acrylates, les tétrahydroperfluoroalkylacrylates, le tris(triméthylsilyloxy)propyle méthacrylate, le perfluoro hexyléthylthiocarbonylaminoéthyle méthacrylate, le trifluoroéthyle méthacrylate, l'hexafluoroisopropyle méthacrylate, l'hexafluorobutyle méthacrylate, le trifluoroéthyle méthacrylate,
- les composés prépolymères organosilanes, tels que le α,ω-bisméthacryloxy-propyle polydiméthylsiloxane, les polydiméthylsiloxanes à terminaison(s) vinylique(s), les copolymères diphénylsiloxane-diméthylsiloxane à terminaisons vinyliques, les copolymères trifluoropropylméthylsiloxane à terminaison(s) vinylique(s), les copolymères diéthylsiloxane-diméthylsiloxane à terminaison(s) vinylique(s), les copolymères d'éthylsiloxane diméthylsiloxane à terminaison(s) vinylique(s), les copolymères éthylène-siloxane à terminaison(s) vinylique(s), le méthacrylate de tris(polydiméthylsiloxy)silylpropyle, les (méth)acrylates de polysiloxanylalkyle, les méthacryloxypropyle à terminaison polydiméthylsiloxane, le 1,3-divinyl-1,1,3,3-tétraméthyldisiloxane, le 2-(divinylméthylsilyl)éthyltriéthoxysilane, ou le 1,3-divinyl-1,3-diphényl-1,3-diméthyldisilane,
- les composés mixtes fluorés / silanes ou chlorés / silanes, tels que le fluoro-méthacryloxypropyl tris(triméthylsiloxy) silane, 3-acryloxy propyl Méthyl dichlorosilane, le 3-acryloxypropyltrichlorosilane, le 3-méthacryloxypropyl Trichlorosilane,
- les composés silanes / isocyanates tel que le 3-Isocyanatopropyltriéthoxysilane, et
- les monomères vinyliques, tels que les N-vinyl lactames ; par exemple N-vinyl pyrrolidone (NVP)), N-vinyl-N-méthyl acétamide (VMA), N-vinyl-N-éthyl acétamide, N-vinyl-N-éthyl formamide, N-vinyl formamide, N-2-hydroxyéthyl vinyl carbamate, N-carboxy-alanine N-vinyl ester, de préférence CH₃C(O)N(CH₃)-CH=CH₂ (N-vinyl-N-méthyl acétamide (VMA)).

De préférence, le constituant A) est le 2-hydroxyéthylméthacrylate (HEMA) ou ceux illustrés dans les exemples ci-après.

Le constituant A) peut être présent à hauteur d'environ 0,10 à 99,90% massique dans la composition, par exemple entre 10,00 et 99,00 ou entre 50,00 et 90,00%.

On entend par « polyol », au sens de la présente invention, tout composé organique possédant au moins deux groupes alcool.

On entend par oligoglycérol, au sens de la présente invention, un polymère de glycérol, dont le degré de polymérisation moyen est compris de 2 à 7, l'oligoglycérol pouvant être fonctionnalisé, de structure linéaire, ramifiée ou hyperramifiée.

On entend par « polyglycérol », au sens de la présente invention, un polymère de glycérol, dont le degré de polymérisation moyen est supérieur à 7. Il peut par exemple s'agir d'un polyglycérol ayant une masse molaire comprise entre 546 et 100 000 g/mol, de préférence entre 546 et 20000 g/mol, et plus particulièrement entre 2000 et 5000 g/mol, 546 g/mol n'étant pas inclus.

Le polyglycérol peut être obtenu par toute méthode connue de l'homme du métier, par exemple par la polymérisation d'un monomère amorcée par un amorceur en présence d'un catalyseur, avec ou sans solvant. Le degré de polymérisation moyen doit être strictement supérieur à 7, par exemple compris de 8 à 270, par exemple entre 15 et 70.

On entend par « polyglycérol linéaire », au sens de la présente invention, un polymère de glycérol comprenant un squelette de résidus de glycérol liés de manière linéaire, chaque unité monomère n'étant liée chimiquement par liaison éther qu'à deux autres unités monomères.

On entend par « polyglycérol ramifié », au sens de la présente invention, un polymère de glycérol constitué d'unités glycérols reliées par des liaisons éther, dont la masse molaire peut être comprise entre 546 et 100 000 g/mol, par exemple entre 546 et 10 000 g/mol, ou entre 800 et 6000 g/mol, 546 g/mol n'étant pas inclus. Le polyglycérol ramifié peut présenter des degrés de polymérisation (DP) par exemple supérieur ou égal à 8, des dispersités comprises entre 1,1 et 5, par exemple entre 1,1 et 1,8 et des degrés de ramification (ou degrés de branchement (DB)) selon la définition de Frey et al. (Acta Polym. 1997,48, 30 **([3])**), compris entre 0,05 et 0,3.

On entend par polyglycérol hyperramifié, au sens de la présente invention, un polymère de glycérol constitué d'unités glycérols reliées par des liaisons éther, dont la masse molaire peut être comprise entre 546 et 100 000 g/mol, par exemple entre 546 et 10 000 g/mol, ou entre 800 et 6000 g/mol, 546 g/mol n'étant pas inclus. Le polyglycérol hyperramifié peut présenter des degrés de polymérisation (DP) par exemple supérieur à 8, des dispersités comprises entre 1,1 et 5 et des degrés de ramification (ou degrés de branchement (DB)) selon la définition de Frey et al. **([1])** compris entre 0,3 et 0,8, et pouvant même aller jusqu'à 1,0 en dopant le DB au moyen d'une modification post-polymérisation. Par exemple, le polyglycérol hyperramifié peut être un dendrimère de polyglycérol ou un polyglycérol hyperramifié de degré de ramification compris entre 0,3 et 0,7, ou un mélange de polyglycérol hyperramifié et d'oligoglycérols ou de polyglycérols linéraires Le polyglycérol hyperramifié peut être obtenu par toute technique connue de l'homme du métier, par exemple par la méthode décrite par Sunder et al. (« Controlled Synthesis of Hyperbranched Polyglycerols by Ring-Opening Multibranching Polymerization », Macromolecules 1999, 32, 13, 4240-4246 **([5])**) pour obtenir des masses molaires moyennes allant jusqu'à 6000 g/mol et par la méthode décrite par Kainthan et al. (« Synthesis, Characterization, and Viscoelastic Properties of High Molecular Weight Hyperbranched Polyglycerols », Macromolecules 2006, 39, 22, 7708-7717 **([6])**) pour l'obtention de masses molaires moyennes supérieures à 6000g/mol.

Les masses molaires moyennes en nombres et en masse du polyglycérol hyperramifié peuvent être déterminées par chromatographie d'exclusion stérique (SEC) et/ou spectroscopie RMN ¹H.

D'une manière générale, le degré de ramification (DB) peut être déterminé en utilisant les méthodes classiques de l'état de l'art, par exemple par RMN ¹³C Inverse Gated.

On entend par « dendrimère », au sens de la présente invention, une molécule constituée de 1 ou plusieurs dendrons émanents d'une unique unité constituante, un dendron étant une molécule présentant une seule valence libre ou unité focale, comprenant exclusivement des unités répétitives constitutives de nature dendritiques et terminales, dans laquelle chaque trajet de la valence libre (unité focale) à une quelconque des unités terminales comprend le même nombre d'unités répétitives constitutives. Un dendrimère de polyglycérol est une macromolécule monodisperse de glycérol, dont la forme reprend celle des branches d'un arbre, et dont la structure est symétrique.

Le constituant B) peut être présent à hauteur de 0,1 à 99,99% massique de la composition réticulable.

Le polyglycérol hyperramifié peut s'obtenir par toute méthode connue de l'homme du métier, par example par polymérisation par ouverture de cycle du glycidol ou bien du glycérol carbonate, en utilisant un ou plusieurs amorceurs mono ou polyfonctionnels comme par exemple :
- des monoalcools tels que le méthanol, le butanol, le phénol et ses dérivés, l'alcool benzylique, le 1-dodécanol, le 1-tétradécanol, le 1-hexadécanol, les glycols monoalkyléthers tels que le glycol monoéthyl éther ou le polyéthylène glycol monoalkyléthers, comme par exemple le polyéthylène glycol monoéthyl éther,
- des diols tels que l'éthylène glycol, le diéthylène glycol, le triéthylèneglycol, les polyéthylènes glycols, le 1,2-propanediol, le 1,3-propanediol, le 1,2-butanediol, le 1,3-butanediol, le 2,3-butanediol, 1,2-pentanediol, 1,3-pentanediol, le 1,4-pentanediol, le 1,5-pentanediol, le 2,3-pentanediol, le 2,4-pentanediol, le 1,2-hexanediol, le 1,3-hexanediol, le 1,4-hexanediol, le 1,5-hexanediol, le 1,6-hexanediol, 2,5-hexanediol, le 1,2-heptanediol, le 1,7-heptanediol, le 1,8-octanediol, le 1,2-octanediol, le 1,9 nonanediol, le 1,10-décanediol, le 1,2-décanediol, 1,12-dodécanediol, le 1,2-dodécanediol, le 1,5-hexadiène-3,4-diol, les cyclopentanediols, les cyclohexanediols, l' inositol et ses dérivés, le (2)-méthyl-2,4-pentanediol, le 2,4-diméthyl-2,4-pentanediol, le 2-éthyl-1,3-hexanediol, le 2,5-diméthyl-2,5-hexanediol, le 2,2,4-triméthyl-l ,3-pentanediol, le pinacol, le dipropylène glycol, le 1,4-butanediol, l'hexaméthylène glycol, le bisphénol A, le bisphénol F, le bisphénol S, le diéthylèneglycol, le triéthylène glycol, dipropylène glycol, tripropylène glycol, les polyéthylène glycols HO(CH₂CH₂O)ₙ-H ou les polypropylène glycols HO(CH[CH₃]CH₂O)ₙ-H (où n est un entier supérieur ou égal à 4) ou des mélanges de deux ou plus des composés présentés ci-dessus,
- des triols ou polyols de fonctionnalité supérieure à 3 tels que le glycérol, le diglycérol, le triglycérol, le tétraglycérol, le triméthyloléthane, le triméthylolpropane, le di-triméthylolpropane, le sorbitol,
- des oligomères à terminaisons hydroxyles tels que les pentaérythritols éthoxylés et les pentaérythrithols propoxylés, les triméthylolpropanes éthoxylés et les triméthylolpropanes propoxylés, les glycérols éthoxylés ou propoxylés, résultant de la réaction d'addition par ouverture de cycle de l'oxyde d'éthylène et/ou de l'oxyde de propylène sur le pentaérythritol, le triméthylolpropane et le glycérol. Le degré d'éthoxylation ou respectivement de propoxylation est habituellement compris entre 0,1 et 10 unités d'oxyde d'éthylène ou respectivement d'oxyde de propylène par fonction OH. La masse molaire est généralement comprise entre 100 et 1000 g/mol. On peut utiliser de préférence les triméthylolpropanes éthoxylés, les glycérols éthoxylés et les pentaérythritol éthoxylés,
- des molécules « étoilées » possédant au moins trois bras comprenant des blocs Polyoxypropylène-polyoxyéthylène peuvent aussi être utilisées telles que les sorbitols et saccharides éthoxylés ou propoxylés, l'amidon dégradé, le polyvinylalcool.
- des amorceurs tels que l'eau, la méthylamine, l'éthylamine, la propylamine, la butylamine, la dodécylamine, la myristylamine, la palmitylamine, la stéarylamine, l'aniline, la benzylamine, l'ortho ou la para-toluidine, l'α,β-naphtylamine, l'ammoniaque, l'éthylène diamine, la propylène diamine, la 1,4 butylène diamine, les 1,2-, 1,3-, 1,4-, 1,5-, ou 1,6-hexaméthylène diamines, ainsi que les o-,m- et p-phénylènes diamines, les 2,4- et 2,6-tolylènediamine, les 2,2'-, 2,4 and 4,4`-diaminodiphénylméthane, les 2,2'-, 2,4 and 4,4'-diaminodicyclohexyleméthane, la diéthylène glycol diamine, la diéthylène triamine, la triétylène tétramine, les poly(propylèneglycol)diamines (Jefifamines) difonctionnelles ou trifonctionnelles,
- des amino-alcools tels que la diéthanolamine, la dipropanolamine, la diisopropanolamine, la triéthanolamine, le tris(hydroxyméthyl)aminométhane ou la diisopropyléthanolamine,
- des composés contenant des groupements fonctionnels tels que l'allylalcool, l'allylglycérol, le 10-undécèneol, la 10-undécèneamine, la dibenzylamine.

L'amorceur peut être ensuite partiellement déprotoné par un agent approprié, par exemple choisi parmi les métaux alcalins et leurs hydrures, les alkoxydes, et les hydroxydes. De manière préférentielle, les métaux ou les alkoxydes de métaux comme le méthanolate de potassium (MeOK) sont utilisés. Le carbonate de potassium peut également être utilisé comme catalyseur de la polymérisation du carbonate de glycérol notamment.

D'autres molécules peuvent être utilisées pour catalyser la polymérisation, comme par exemple les alcoolates, les organométalliques, les sels de métaux, les amines tertiaires. Parmi les alcoolates, on utilisera les alcoolates de métaux alcalins tels le méthylate de sodium, l'isopropylate de potassium, le méthanolate de potassium. On peut aussi utiliser les hydroxydes de tétraalkylammonium, tels les hydroxydes de tétraméthylammonium; les hydroxydes de métaux alcalins, tels l'hydroxyde de sodium et l'hydroxyde de potassium, les sels de métaux, tels les composés organiques et/ou inorganiques à base de fer, plomb, bismuth, zinc et/ou l'étain à des niveaux d'oxydation de métaux conventionnels par exemple : chlorure de fer(II), chlorure de fer(III), bismuth(lll) 2-éthylhexanoate, bismuth(lll) octoate, bismuth(lll) néodécanoate, chlorure de zinc, zinc 2-éthylcaproate, octoate d'étain (II), éthylcaproate d'étain(II), palmitate d'étain (II), dibutyldilaurate d'étain (IV) (DBTL), dibutyldichlorure d'étain (IV) ou octaote de plomb; les amidines, telles la 2,3-diméthyl-3,4,5,6-tétrahydropyrimidine.

On peut aussi utiliser les sels de métaux alcalins d'acides gras à longue chaîne possédant 10 à 20 atomes de carbone et optionnellement des groupements pendants OH. Enfin, les amines tertiaires telles que la triéthylamine, tributylamine, diméthylbenzylamine, diéthylbenzylamine, pyridine, méthylpyridine, dicyclohexylméthylamine, diméthylcyclohexylamine, N,N,N',N'-tétraméthyldiaminodiéthyl éther, bis (diméthylaminopropyl)-urée, N-méthyl- et N-éthylmorpholine, N-cocomorpholine, N-cyclohexylmorpholine, N,N,N',N'-tétraméthyléthylènediamine, N,N,N',N' tétraméthyl- 1 ,3 -butanediamine, N,N,N' ,N' - tétraméthyl -1 ,6 - hexanediamine, pentaméthyldiéthylènetriamine, N-méthylpipéridine, N-diméthylaminoéthylpipéridine, N,N'-diméthylpipérazine, N-méthyl-N'-diméthylaminopipérazine, 1,8-diazabicyclo(5.4.0)undéc-7-ène (DBU), le TBD (1,5,7-triazabicyclo[4.4.0]déc-5-ène), 1,2 diméthylimidazole, 2-méthylimidazole, N,N-diméthylimidazole-[3-phényléthylamine, le DABCO ou 1,4-diaZabicyclo-(2,2,2)octane, bis-(N,N-diméthylaminoéthyl)adipate; composés de type alcanolamine, tels la triéthanolamine, triisopropanolamine, N-méthyl- et N-éthyl-diéthanolamine, diméthylaminoéthanol, 2-(N,N-diméthylaminoéthoxy)éthanol, les N,N',N" tris-(dialkylaminoalkyl)hexahydrotriazines, tels la N,N',N" tris-(diméthylaminopropyl)-s-hexahydrotriazine et/ou le (diméthylaminoéthyl)éther.

La réaction peut avoir lieu en présence de solvant, par exemple un solvant aliphatique, cyclo aliphatique ou aromatique comme la décaline, le toluène ou le xylène, ou un éther comme le glyme, le diglyme ou le triglyme). Alternativement, la réaction peut avoir lieu en masse, par exemple entre 40 et 140°C, de préférence à 95°C en semi-batch, par addition lente et contrôlée des monomères au milieu réactif.

Le polyglycérol hyperramifié peut s'obtenir également par co-polymérisation avec d'autres monomères fonctionnalisés pouvant incorporer au moins un groupement choisi parmi des fluorés, des silanes, des siloxanes, et des halogénés tels que l'oxyde de propylène, l'oxyde d'éthylène, l'oxyde de butylène, l'épichlorhydrine, le vinyloxirane, le glycidyl allyl éther, le glycidyl méthacrylate, l'isopropyl glycidyl éther, le phénylglycidyl éther, le 2-éthylhexyl glycidyl éther, l'hexadécyl glycidyl éther, le naphthyl glycidyl éther, le t-butyldiméthylsilyl-(R)-(-)-glycidyl éther, le benzyle glycidyl éther, l'époxy-3-phénoxypropane, le biphénylyl glycidyl éther, le propargyl glycidyl éther, les n-alkyl-glycidyl éthers, mais aussi des oxiranes fonctionnalisés tels que le γ-glycidylpropyltriméthoxysilane, le γ-glycidylpropyltriéthoxysilane, le γ-glycidoxypropyl-bis(triméthylsiloxy)-méthylsilane et le 3-(Bis(triméthylsiloxy)méthyl)-propyl glycidyl éther, le glycidylglycérol éther, le glycidyl butyléther, le glycidylnonylphényléther, les oxiranes fluorés et perfluorés tels que l'hexafluoropropylène oxyde, 2,3-difluoro-2,3-bis-trifluorométhyl-oxirane, 2,2,3-trifluoro-3-pentafluoroéthyl-oxirane, 2,3-difluoro-2-(1,2,2,2-tétrafluoro-1 -trifluorométhyl-éthyl)-3-trifluorométhyl-oxirane, 2-fluoro-2-pentafluoroéthyl-3,3-bis-trifluorométhyl-oxirane, 1,2,2,3,3,4,4,5,5,6-décafluoro-7-oxa-bicyclo[4.1.0]heptane, 2,3-difluoro-2-trifluorométhyl-3-pentafluoroéthyl-oxirane, 2,3-difluoro-2-nonafluorobutyl-3-trifluorométhyl-oxirane, 2,3-difluoro-2-heptafluoropropyl-3-pentafluoroéthyl-oxirane, 2-fluoro-3-pentafluoroéthyl-2,3-bis-trifluorométhyl-oxirane, 2,3-bis-pentafluoroéthyl-2,3-bistrifluorométhyl-oxirane, 2,3-bis-trifluorométhyl-oxirane, 2-pentafluoroéthyl-3-trifluorométhyl-oxirane, 2-(1,2,2,2-tétrafluoro-1-trifluorométhyl-éthyl)-3-trifluorométhyl-oxirane, 2-nonafluorobutyl-3-pentafluoroéthyl-oxirane, 2,2-bis-trifluorométhyl-oxirane, 2-heptafluoroisopropyloxirane, 2-heptafluoropropyloxirane, 2-nonafluorobutyloxirane, 2-tridécafluorohexyloxirane, 2-pentafluoroéthyl-2-(1,2,2,2-tétrafluoro-1 -trifluorométhyl-éthyl)-3,3-bis-trifluorométhyl-oxirane, 2-fluoro-3,3-bis-(1,2,2,2-tétrafluoro-1-trifluorométhyl-éthyl)-2-trifluorométhyl-oxirane, 2-fluoro-3-heptafluoropropyl-2-(1,2,2,2-tétrafluoro-1-trifluorométhyl-éthyl)-3-trifluorométhyl-oxirane and 2-(1,2,2,3,3,3-hexafluoro-1-trifluorométhyl-propyl)-2,3,3-tris-trifluorométhyl-oxirane.

Ces types de composés ou groupements peuvent être introduits *in situ* par copolymérisation ou par post-modification.

De préférence, le polyglycérol est un polyglycérol hyperramifié.

Le constituant B), notamment lorsqu'il s'agit de polyglycérol hyperramifié fonctionnalisé, peut être présent dans la composition en tant qu'agent de réticulation, en tant que co-monomère ou bien en tant que monomère principal. Une fois fonctionnalisé, par exemple avec des fonctions vinyliques ou isocyanatoalkyl(méth)acrylates tels, que le 2-isocyanatoéthylméthacrylate et le 2-isocyanatoéthyl acrylate, maléate, fumarate, itaconate, citraconates et vinyles réactives, il peut être utilisé comme agent de réticulation. Les monomères fonctionnels peuvent également permettre l'amélioration des propriétés physico-chimiques du matériau tel des monomères de types siloxanes, silanes, fluorés.

La fonction C=C polymérisable sur le polyglycérol hyperramifié fonctionnalisé peut être choisie parmi les groupements isocyanatoalkyl(méth)acrylate, maléiques, fumariques, itaconiques, ou vinyliques de structure: 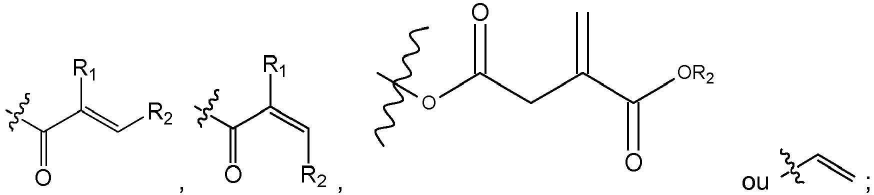 dans laquelle R₁ représente H ou méthyle ; R₂ représente H, C₁₋₆alkyle linéaire ou ramifié, ou -C(=O)OR_{2A} dans lequel R_{2A} représente H ou C₁₋₆alkyle linéaire ou ramifié éventuellement substitué par un ou plusieurs groupements hydroxyles, et R₃ représente H, C₁₋₂₀ alkyle linéaire ou ramifié.

Le groupement portant une fonction C=C polymérisable sur le polyglycérol hyperramifié fonctionnalisé peut être choisi parmi: 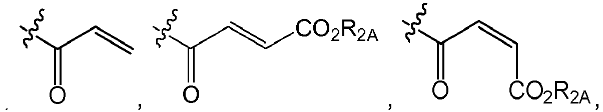 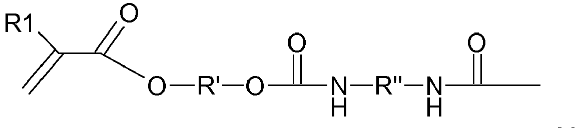 où :
- R1 = H ou CH₃
- R_{2A} représente H ou C₁₋₆alkyle linéaire ou ramifié substitué par un ou plusieurs groupements hydroxyles, de préférence 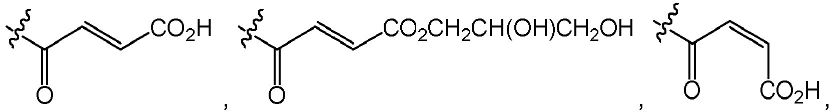 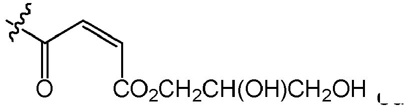
- R' et R" représentent indépendamment un radical alkylène linéaire ou ramifié, saturé ou insaturé, substitué ou non, ayant de 2 à 20 atomes de carbone, un radical cycloalkylène saturé ou insaturé, substitué ou non, ayant de 3 à 20 atomes de carbone, un radical arylène substitué ou non ayant de 6 à 20 atomes de carbone, un radical arylène-alkylène radical ayant de 7 à 20 atomes de carbone, un radical hétérocyclique ou toute séquence linéaire ou ramifiée de deux ou plusieurs des radicaux mentionnés, éventuellement liés par l'intermédiaire d'une structure éther, thioéther, ester, amine ou amide.

La fonctionnalisation peut se faire par toute méthode connue de l'homme du métier, par exemple en semi-batch par addition lente et contrôlée de l'agent de fonctionnalisation et en présence ou non d'un solvant polaire, sous gaz inerte, de préférence à une température comprise entre 25 et 200°C et plus particulièrement entre 60 et 90°C. Pour être utilisé en tant qu'agent de réticulation, le polyol, par exemple le polyglycérol, doit présenter une fonctionnalisation moyenne supérieure à 1, de préférence supérieure ou égale à 2, et peut aller jusqu'à 100% en nombre d'OH présent sur la molécule de polyglycérol hyperramifié.

Par exemple, le polyglycérol pur ou le copolymère de glycidol et d'un autre monomère oxirane peut être fonctionnalisé par réaction d'addition ou condensation sur les fonctions OH par un agent choisi parmi:
- l'anhydride maléique et les dérivés de l'acide maléique tels que les maléates et fumarates. Il peut s'agir par exemple du diméthylmaléate, du diéthylmaléate, ou des esters de maléate supérieurs où R est un groupement alkyle contenant 3 à 20 atomes de carbone, de l'acide fumarique, du diméthylfumarate, du diéthylfumarate ou des esters de fumarate supérieurs où R est un groupement alkyle contenant 3 à 20 atomes de carbone. La réaction de fonctionnalisation avec l'anhydride peut être réalisée entre 0°C et 120°C. La réaction avec l'acide maléique ou les maléates ou fumarates peut être réalisée à température comprise entre 90°C et 200°C en présence ou non de catalyseurs d'estérification ou de trans-estérification et dans des conditions connues de l'homme de l'art,
- l'anhydride itaconique et ses dérivés, tels que l'acide itaconique, le diméthylitaconate, ou les esters d'itaconate supérieurs, où R est un groupement alkyle contenant 3 à 20 atomes de carbone. La réaction de fonctionnalisation avec l'anhydride peut être réalisée entre 0°C et 120°C. La réaction avec l'acide itaconique ou les itaconates peut être réalisée à une température comprise entre 90°C et 200°C, en présence ou non de catalyseurs d'estérification ou de trans-estérification et dans des conditions connues de l'homme de l'art,
- l'anhydride citraconique et ses dérivés tels que l'acide citraconique, le diméthylcitraconate, ou les esters de citraconate supérieurs, où R est un groupement alkyle contenant 3 à 20 atomes de carbone. La réaction de fonctionnalisation avec l'anhydride est réalisée entre 0°C et 120°C. La réaction avec l'acide citraconique ou les citraconates est réalisée à température comprise entre 90°C et 200°C, en présence ou non de catalyseurs d'estérification ou de trans-estérification et dans des conditions connues de l'homme de l'art,
- les isocyanatoalkyl(méth)acrylates tels, que 2-isocyanatoéthylméthacrylate, 2-isocyanatoéthyl acrylate. Ces isocyanato-alkyl(méth)acrylates sont disponibles commercialement, ou peuvent aussi être synthétisés selon la méthodologie décrite dans le document US20010005738 **([1])**. La fonctionnalisation du polyglycérol par un isocyanatoalkyl(méth)acrylate conduit au groupement suivant : 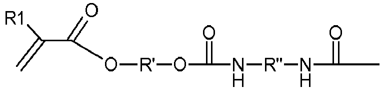 où :
   - R1 = H ou CH₃
   - R' et R" représentent indépendamment un radical alkylène linéaire ou ramifié, saturé ou insaturé, substitué ou non, ayant de 2 à 20 atomes de carbone, un radical cycloalkylène saturé ou insaturé, substitué ou non, ayant de 3 à 20 atomes de carbone, un radical arylène substitué ou non ayant de 3 à 20 atomes de carbone, un radical arylènealkylène ayant de 4 à 20 atomes de carbone, un radical hétérocyclique ou toute séquence linéaire ou ramifiée de deux ou plusieurs des radicaux mentionnés, éventuellement liés par l'intermédiaire d'une structure éther, thioéther, ester, amine ou amide.

De préférence, dans le constituant B), la fonction C=C polymérisable de type isocyanatoalkyl(méth)acrylate, est par exemple choisi parmi le 2-isocyanatoéthylméthacrylate et le 2-isocyanatoéthyl acrylate, et leur mélange.

Dans le cas des anhydrides générant une fonction acide après réaction avec une fonction OH du polyglycérol, ou dans le cas de fonctionnalisation avec un diacide, la fonction acide carboxylique résiduelle peut ensuite réagir avec une fonction époxy d'un composé de type époxysilane, époxyfluoré, glycidyl(méth)acrylate, monoépoxy tels que le glycidol, les oxiranes pouvant être utilisés comme co-monomères tels que définis ci-avant, ou tout autre monoépoxy de formule :

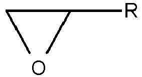

Cette réaction conduit à la formation d'une liaison ester par réaction sur la fonction acide carboxylique. La nature du radical R permettra d'ajuster les propriétés du polyol, notamment du polyglycérol.

Alternativement, des réactions d'éthérification de Williamson peuvent être mises en jeu par réaction d'halogénures d'allyle sur des fonctions hydroxyle, mais aussi par alkylation par transfert de phase en présence de soude comme décrit dans Journal of The American Society (2000) 122, 2954-2955 **([4]).**

Dans un autre mode de réalisation, le polyglycérol hyperramifié fonctionnalisé par au moins une insaturation, préférentiellement 2 insaturations, peut être additionnellement fonctionnalisé par des groupements choisis parmi des groupements fluorés, silicones et silanes, par réaction de monomères tels que décrits ci-avant en relation avec le constituant A). Dans ce mode de réalisation, il peut s'agir d'une réaction directe de la fonction époxy sur les groupements OH terminaux, soit avant, soit après la fonctionnalisation par des groupements insaturés. Alternativement, il peut s'agir d'une réaction sur la fonction acide carboxylique générée par la réaction de fonctionnalisation par les anhydrides maléiques, itaconiques et citraconiques notamment.

Concernant le constituant C), cet amorceur peut être un amorceur UV, thermique ou redox, tel que :
- un amorceur photochimique de la famille des hydroxybenzophénones tels que la 2-hydroxy-4-acryloyloxyéthoxy benzophénone, la 2-hydroxy-4-(2 hydroxy-3-méthacrylyloxy) propoxybenzophénone ; la benzophénone, l'acétophénone, l'acétonaphthoguinone, la méthyl éthyl cétone, la valérophénone, l'hexanophénone, la C-phénylbutyrophénone, la p-morpholinopropiophénone, la dibenzosubérone, la 4-morpholinobenzophénone, la 4-morpholinodéoxybenzoïne, le p-diacétylbenzène, la 4-aminobenzophénone, la para-méthoxyacétophénone, la 3-méthylanthraquinone, la tert-butylanthraquinone, les esters d'anthraquinone, le benzaldéhyde, le 9-acétylphénanthrène, le 2-acétylphénanthrène, la 10-thioxanthénone, le 3-acétylphénanthrène, le 3-acétylindole, la 9-fluorénone, la 1-indanone, le 1,3,4-triacétylbenzène, la thioxanthèn-9-one, la xanthèn-9-one, la 2,4-diméthylthioxanthone, la 2,4-diéthylthioxanthone, la 2,4-di-iso-propylthioxanthone, la 2,4-dichlorothioxanthone, la benzoïne, la benzoïne isobutyléther, la benzoïne tétrahydropyranyléther, la benzoïne méthyléther, la benzoïne éthyléther, la benzoïne butyléther, la benzoïne isopropyléther, la chloroxanthenone, la 7-H- benzoïne méthyl éther, la benzodéanthracèn-7-one, le 1-naphthaldéhyde, la 4,4'-bis(dimethylamino)benzophénone, la 4-phénylbenzophénone, la 4-chlorobenzophénone, la 4,4'-bis(diméthylamino)benzophénone, la 1-acétonaphthone, la 2-acétonaphthone, le 1-benzoylcyclohexan-1-ol, la 2-hydroxy-2,2-diméthylacétophénone, la 2.2-diméthoxy-2-phénylacétophénone, la 2,2-diéthoxy-2-phénylacétophénone, la 1,1-dichloroacétophénone, la 1-hydroxyacétophénone,l'acétophénone diméthyl acétal, l'o-méthoxybenzophénone,la triphénylphosphine, la tris-o-tolylphosphine, la benzaanthracène-7,12-dione, la 2,2-diéthoxyacétophénone, les benzyl acétals tels que le benzyl diméthyl acétal, la 2-méthyl-1-4-(méthylthio)phényl-2-morpholinopropan-1-one, les anthraquinones telles que la 2-méthylanthraquinone, la 2-éthylanthraquinone, la 2-tert-butylanthraquinone, la 1-chloroanthraquinone, la 2-amylanthraquinone, la 2,3-butanedione,
- des photo-amorceurs peu ou non jaunissants tels que les esters de l'acide phénylglyoxalique, des combinaisons des différents photo-amorceurs tels que mentionnés précédemment, et les photo-amorceurs en combinaison des promoteurs de photopolymérisation tels que l'acide benzoïque ou des amines,
- un amorceur thermique de type nitrile tel que le 2,2-azobis(2,4-diméthylpentanenitrile), le 2,2'-azobisisobutyronitrile, le 1,1'-azobis(cyclohexanecarbonitrile), ou le 2,2'-azobis(2-methylpropionitrile), un amorceur thermique de type péroxyde tel que le péroxodisulfate de potassium, le péroxyde de dibenzoyle, le péroxyde de cyclohexanone, le péroxyde di-tert-butyle, le péroxyde d'acétyle cyclohexylsulfonyle, le percarbonate de diisopropyle, le péroctoate ou benzopinacol de tert-butyle, le péroxyde de di-t-butyle, l'hydropéroxyde de cumène,le péroxyde de dicumyle,le perbenzoate de t-butyle, ou une amine N-oxyde hydroxylée, comme le 2,2,6,6-tétraméthylpipéridine-N-oxyl et le 4-hydroxy-2,2,6,6-tétraméthylpipéridine-N-oxyl,
- un système d'amorceur rédox permettant de conduire les réactions de polymérisation à faible température tel que des oxydants dont les dérivés azo tels que l'AIBN, les péroxydes comme par exemple le péroxyde d'hydrogène, le péroxyde de benzoyle, le t-butyl-hydropéroxyde, l'hydropéroxyde de p-mentane, le persulfate d'ammonium, le persulfate de sodium, le persulfate de potassium, des réducteurs tels que l'acide ascorbique, le bisulfite de sodium, la N,N,N',N'-tétraméthyléthylène diamine le formaldéhyde sulfoxylate de sodium, les sels de métaux Fe²⁺, Cr²⁺, Zn²⁺, V²⁺, Ti³⁺, Co²⁺, Cu⁺ comme par exemple le sulfate de Fer FeSO₄ notamment, ainsi que les N,N-dialkylanilines tels que la N,N-diméthyl-p-toluidine.

Les agents filtrants peuvent être choisis parmi les filtres de protection de l'œil contre les rayonnements du domaine du visible ou proche du visible tels que les filtres UV, les absorbants UV, ou les filtres de la lumière bleue, les composés photochromiques, et les filtres infra-rouge ou des filtres de lumière visible à bande d'absorption spécifiques. Les agents filtrants adaptés à la mise en oeuvre de la composition réticulable selon l'invention peuvent être tout agent filtrant adapté disponible commercialement, comme les filtres de protection de l'œil contre les rayonnements du domaine du visible ou proche du visible tels que les filtres UV, les absorbants UV, ou les filtres de la lumière bleue, les composés photochromiques, et les filtres infra-rouge ou des filtres de lumière visible à bande d'absorption spécifiques. Il peut s'agir par exemple de l'AEHB (acryloxyéthoxyhydroxybenzophénone). De même, les absorbants UV peuvent être tout absorbant UV présentant une haute absorption dans la gamme UV-A (320-380 nm), mais relativement transparent au-delà de 380 nm. Généralement, si l'absorbant UV est présent dans la composition de l'invention, il l'est entre 0,5 et 1,5% massique des réactifs, par exemple à 1% massique. Avantageusement, les agents anti-UV peuvent être incorporés à l'hydrogel à l'étape d'hydratation post-polymérisation.

Un autre objet de l'invention se rapporte à une composition réticulée susceptible de former un polymère hydrogel, issue de la réticulation de:
A) au moins un monomère polymérisable ou un mélange de monomères polymérisables ou un mélange de monomères et de macromonomères polymérisables portant au moins une fonction C=C polymérisable de type acrylate, méthacrylate, maléate, fumarate, itaconate, citraconate, styrénique ou vinylique ;
B) au moins un polyglycérol linéaire, ramifié ou hyperramifié ou un mélange de polyglycérol hyperramifié et de polyglycérols linéaire, ramifié ou hyperramifié comprenant en moyenne plus de 1 groupement hydroxyle fonctionnalisé avec un groupement portant une fonction C=C polymérisable de type acrylate, méthacrylate, maléate, fumarate, itaconate, citraconate, styrénique ou vinylique ;
C) un amorceur radicalaire ou un mélange d'amorceurs radicalaires susceptible d'amorcer la polymérisation par voies thermiques, rédox ou photochimiques; et
D) éventuellement au moins un additif choisi parmi les antioxydants, les agents permettant d'ajuster les propriétés physico-chimiques telles que le module d'Young, l'élongation et la contrainte à la rupture, les agents de promotion, de modulation et /ou de contrôle de la perméabilité à l'oxygène, les plastifiants, les agents humectants, les lubrifiants, les agents de modification de la viscosité, les agents compatibilisants, les agents de coloration, les agents filtrants, les agents thérapeutiques, les agents anti-microbiens et les anti-biofilms bactériens.

Les constituants A), B), C) et D) sont tels que décrits précédemment dans le cadre de la composition réticulable.

La formation d'un gel peut être obtenue par une composition réticulée de l'invention, comprenant le constituant A), le constituant B) à hauteur de 0,10 à 99,99% massique de la composition réticulée et le constituant C) présent entre 0,05 et 5% massique.

Un autre objet de l'invention se rapporte à un hydrogel susceptible d'être obtenu par :
- hydratation/gonflement à partir d'eau ou d'une solution aqueuse d'une composition réticulée telle que définie ci-avant, ou,
- polymérisation de la composition réticulable en présence d'un solvant protique ou aprotique miscible à l'eau, puis échange du solvant avec un excès d'eau ou une solution aqueuse, ou
- par polymérisation de la composition réticulable en présence d'eau ou d'une solution aqueuse.

L'étape d'hydratation/gonflement peut être réalisée par l'homme du métier pendant un temps adapté au gonflement requis. Elle peut être réalisée par exemple pendant 12h, à température ambiante (soit environ 25°C).

Avantageusement, la solution aqueuse peut contenir un composé d'intérêt choisi parmi les principes actifs thérapeutiques, les vitamines ou les produits lubrifiants. Il peut s'agir par exemple de tocophérols, qui sont des anti-oxydants très puissants pour le traitement de l'uvéite antérieure chronique, ou de l'acide hyaluronique, pour le traitement ou la prévention de la sécheresse oculaire.

Un autre objet de l'invention se rapporte à un procédé de préparation d'une composition réticulée de l'invention, consistant à faire réagir, en présence ou en l'absence de solvant aprotique, de préférence en l'absence de solvant :
A) au moins un monomère polymérisable ou mélange de monomères polymérisables ou un mélange de monomères et de macromonomères polymérisables portant au moins une fonction C=C polymérisable de type acrylate, méthacrylate, maléate, fumarate, itaconate, citraconate, styrénique ou vinylique ;
B) au moins un polyol choisi parmi les oligoglycérols, les polyglycérols linéaires, ramifiés et hyperramifiés, ou un mélange de polyglycérol hyperramifié et d'un polyol choisi parmi les oligoglycérols et les polyglycérols linéaires, ramifiés et hyperramifiés, comprenant en moyenne plus de 1 groupements hydroxyles fonctionnalisés avec un groupement portant une fonction C=C polymérisable de type acrylate, méthacrylate, maléate, fumarate, itaconate, citraconate, styrénique ou vinylique ;
C) au moins un amorceur radicalaire ou un mélange d'amorceurs radicalaires pouvant amorcer la polymérisation par voies thermiques, rédox ou photochimiques; et
D) éventuellement au moins un additif choisi parmi les antioxydants, les agents permettant d'ajuster les propriétés physico-chimiques telles que le module d'Young, l'élongation et la contrainte à la rupture, les promoteurs de la perméabilité à l'oxygène, les plastifiants, les agents humectants, les lubrifiants, les agents de modification de la viscosité, les agents compatibilisants, les agents de coloration, les agents filtrants, les agents anti-microbiens, les agents thérapeutiques et les anti-biofilm bactérien.Les constituants A), B), C) et D) sont tels que décrits précédemment dans le cadre de la composition réticulable.

Dans ce procédé :
- le solvant aprotique, lorsqu'il est utilisé, peut être choisi parmi les solvants polaires aprotiques tels que le chloroforme, le diméthylformamide, le dichlorométhane, l'acétonitrile, et le diméthylsulfoxyde, ou un mélange d'au moins deux de ceux-ci,
- le solvant protique, lorsqu'il est utilisé, peut être choisi parmi l'eau, le méthanol et l'éthanol.

Dans ce procédé, le constituant B), notamment lorsqu'il s'agit de polyglycérol hyperramifié fonctionnalisé, peut être présent à raison de 0,1 à 99,99% massique du poids total des constituants A) à D).

Dans ce procédé, l'amorceur peut être présent à raison de 0,02 à 5% massique, de préférence 0,02 à 2%, du poids total des constituants A) à D).

Avantageusement, ce procédé peut comprendre en outre une étape de moulage de la composition réticulée. L'étape de moulage peut être réalisée par moulage par coulée, moulage en bloc afin d'être usiné par découpe par tournage, coulage par centrifugation ou production additive, de préférence sous atmosphère inerte.

Le procédé peut être réalisé à une température de 0°C à 150°C, de préférence de 0°C à 120°C. Parmi cette plage de températures, les plus basses, soit de 0°C à environ 60°C, concernent les systèmes rédox. Les photopolymérisations ont, elles, lieu à température ambiante ou légèrement supérieure, par exemple entre 15°C et 39°C. A partir de 40°C, on peut utiliser des systèmes d'amorçage thermiques.

Le procédé peut en outre comprendre une étape de recuit, de préférence réalisée à une température de 20 à 200°C, de préférence de 60 à 160°C. Cette étape peut être réalisée pendant un temps adapté, par exemple compris de 1 minute à 48h.

Le procédé peut en outre comprendre une étape :
- d'hydratation/gonflement à partir d'eau ou d'une solution aqueuse d'une composition réticulée selon l'invention, ou,
- de polymérisation de la composition réticulable selon l'invention en présence d'un solvant protique ou aprotique miscible à l'eau, puis échange du solvant avec un excès d'eau ou une solution aqueuse, ou
- de polymérisation de la composition réticulable selon l'invention en présence d'eau ou d'une solution aqueuse.

Comme indiqué précédemment, la solution aqueuse peut contenir un composé d'intérêt choisi parmi les principes actifs thérapeutiques, les vitamines, les nutriments, les agents décontaminants ou les produits lubrifiants comme par exemple l'acide hyaluronique.

Un autre objet de l'invention se rapporte à un article susceptible d'être obtenu par un procédé de préparation d'une composition réticulée tel que défini précédemment. L'article peut être un dispositif médical, tel que une lentille de contact ou intraoculaire, un patch, un implant ou un pansement.

Un autre objet de l'invention se rapporte à une utilisation d'une composition ou d'un hydrogel selon l'invention tels que définis précédemment, pour la fabrication d'un dispositif médical, tel que une lentille de contact ou intraoculaire, un patch, un implant ou un pansement.

Un autre objet de l'invention se rapporte à l'utilisation d'une composition ou d'un hydrogel selon l'invention tels que définis précédemment, comme vecteur d'un composé d'intérêt choisi parmi les principes actifs thérapeutiques, les vitamines, les nutriments, les agents décontaminants ou les produits lubrifiants.

Un autre objet de l'invention se rapporte à une utilisation d'un polyglycérol hyperramifié comprenant en moyenne plus de un groupement(s), de préférence au moins 2 groupements hydroxyles fonctionnalisés avec un groupement portant une fonction C=C polymérisable, en tant qu'agent de réticulation, co-monomère ou bien monomère principal pour la fabrication d'une lentille de contact ou intraoculaire.

Un autre objet de l'invention se rapporte à l'utilisation d'une composition ou d'un hydrogel selon l'invention, dans des applications biomédicales, cosmétiques, ou sanitaires. Il peut s'agir par exemple d'applications dans des dispositifs chirurgicaux, le relargage de principes actifs, l'ingénierie tissulaire, les pansements, la culture d'organes, les tissus biologiques, les réservoirs d'eau pour les plantes, les diffuseurs de parfum, les dépolluants, les milieux de cultures de bactéries, et les absorbants de liquides dans des garnitures de couches pour bébés, les produits d'hygiène féminine et les produits absorbants pour l'incontinence.

Parmi les principes actifs susceptibles d'être relargués par la composition ou l'hydrogel de l'invention, on peut citer tout principe actif connu, par exemple les antiinflammatoires de toutes classes, comme les AINS ou les corticoïdes, les antibiotiques de toutes classes seuls ou associés, les anti-glaucomateux de toute classes seuls ou associés, les antiallergiques de toutes classes seuls ou associés, les médicaments de traitement de la progression de la myopie de toutes classes, tel que les anticholinergiques, les médicaments de traitement de la presbytie, et plus généralement les traitements des pathologies oculaires incluant l'œil dans son intégralité et ses annexes que sont les paupières, les muscles oculomoteurs, les glandes lacrymales et ses sécrétions et les orbites.

Parmi les substances non médicamenteuses susceptibles d'être relarguées par la composition ou l'hydrogel de l'invention, on peut citer par exemple les vitamines, nutriments tels que les antioxydants, les protecteurs du métabolisme ou les agents décontaminants de la lentille comme les antibiofilms bactériens, les antifongiques, les antiamibiens ou les antiviraux.

D'autres avantages pourront encore apparaître à l'homme du métier à la lecture des exemples ci-dessous, donnés à titre illustratif.

### EXEMPLES

### Liste des acronymes :

AIBN : Azobisisobutironitrile
HPG : Polyglycérol hyperramifié
TMP : Triméthylolpropane
MeOK : Méthanolate de potassium
AM : Anhydride Maléique
AI : Anhydride Itaconique
IEA : Isocyanatoéthylacryate
f : fonctionnalité d'une molécule, i.e. nombre moyen de fonctions réactives par (macro)molécule
%f : pourcentage molaire d'OH fonctionnalisés par (macro)molécule
DMF : N,N-Diméthylformamide
HEMA : Méthacrylate 2-Hydroxyéthyle
EWC : teneur en eau massique dans le gel à l'équilibre en pourcentage (%)

### Détermination des propriétés mise en oeuvre dans les exemples

### Détermination de la perméabilité à l'oxygène (Dk) par polarographie

La méthode polarographique est basée sur un montage classique d'électrochimie comportant 3 électrodes : électrode de travail (WE) en or, contre-électrode (CE) en platine et électrode de référence (RE) Ag/AgCI, immergée dans une solution d'électrolyte (KCI) à 0.1M. L'hydrogel est placé à la surface de l'électrode de travail puis de l'oxygène est injecté dans la cellule électrochimique et la variation de courant est mesurée (oxydation de l'oxygène à la surface de la WE). L'intensité du courant mesurée sera dépendante de la quantité d'oxygène passé au travers de l'hydrogel.

Trois tests sont faits pour chaque échantillon, et la moyenne des trois analyses est retenue.

Le potentiostat utilisé pour ces analyses est un DropSens µSTAT 400 .

### Détermination des propriétés mécaniques

Avec l'utilisation d'une machine de traction (M500-30AT) équipée d'une cellule testometric DBBMTCL 50kg, les propriétés mécaniques déterminées sont : le module d'Young, la contrainte à la rupture et l'élongation à la rupture.

Les dimensions des échantillons hydratés sont normalisées à 3mm de largeur pour 10mm entre les mors, l'épaisseur est mesurée à chaque nouvelle analyse. La précharge est de 0.1N pour une vitesse de déformation équivalente à 20 mm/min le tout à température ambiante. Tous les échantillons ont été évalués au moins 3 fois et des moyennes sur les données calculées par le logiciel WinTest ont été calculées.

### Détermination des propriétés de surface

Les matériaux conçus sont également analysés via différentes techniques incluant des mesures de mouillabilité (a), de rugosité de surface par microscopie à force atomique (AFM) (b), de coefficients de friction au tribomètre (c)

### (a) Mesures de mouillabilité

Les propriétés de surfaces ont également été déterminées par des mesures d'angles de contact de l'eau sur les matériaux en utilisant le mode goutte sur solide sur un appareil KRUSS DSA 100. Brièvement, une goutte (2µL) d'eau distillée est déposée à la surface du matériau et l'angle (en °) à l'équilibre de la goutte avec le matériau est mesuré via une caméra video. Une moyenne de 10 mesures est effectuée via le logiciel DropShapeAnalysis.

### (b) Mesures de rugosité de surface par Microscopie à Force Atomique (AFM)

Les analyses d'AFM ont été effectuées sur un appareillage Dimension EDGE de chez Bruker. Les analyses ont été effectuées en mode Tapping. Des leviers de force 3N/m. avec des pointes Si₃N₄ (Bruker, Ref : RFESP) ont été utilisées pour générer les images de phase, amplitude et hauteur. Les clichés de hauteur nous ont permis d'accéder au R(max), Ra et Rq, définies respectivement comme la hauteur maximale identifiée sur le surface de l'échantillon ; la rugosité de surface moyenne et la déviation standard de la surface plane moyenne. Les échantillons ont été analysés sur des longueurs de 20, 10, 5 et 1 µm pour générer des aires scannées de 400, 100, 25 et 1 µm². Les données ont été traitées avec le logiciel Nanoscope Analysis, et comparées à des lentilles commerciales.

### (c) Mesure de coefficients de friction au tribomètre

Les mesures ont été effectuées sur un tribomètre CSM instrument. Une bille d'acier de diamètre 10 mm a été utilisée à une vitesse de 1cm/sec, avec une force normale de 0.5 N. 3 analyses ont été effectuées sur chaque échantillon et une moyenne des 3 a été calculée. L'échantillon est sous forme de film, et l'analyse a lieu en milieu liquide (eau ou sérum physiologique) à température ambiante.

### Analyses en transmittance UV

La transmittance a été déterminée à l'aide d'un spectrophotomètre UV. Une lentille est placée dans une cuve contenant une solution saline. La cuve est placée dans le compartiment échantillon. Une cuve contenant uniquement la solution saline est placée dans le compartiment référence.

Et le spectre en % transmittance est enregistré entre 200 et 780nm. L'échantillon est analysé 3 fois et une moyenne des 3 mesures à 550nm a été retenue.

### Teneur en eau et taux de gonflement

La teneur en eau et le taux de gonflement sont déterminés en mesurant le poids du gel à l'état sec et à l'état hydraté en utilisant les équations 1 et 2.

Les gels à l'état hydratés sont pesés individuellement après avoir retiré l'excès d'eau à la surface. Les gels sont ensuite séchés dans une étuve à 80°C pendant un minimum de 6h et pesés à nouveau. Ce processus est répété 3 fois, et la valeur d'EWC est la moyenne des 3.

### Exemple A : Synthèse d'un polyglycérol hyperramifié de Mₙ= 2487 g.mol⁻¹

Dans un ballon tricol de 250mL, le triméthylolpropane (TMP ; 1eq, 3.417g) et le méthanolate de potassium à 25% dans le méthanol (MeOK ; 0.3eq, 2.126g) sont introduits.

Le ballon est ensuite placé à l'évaporateur rotatif à 70°C jusqu'à complète dissolution du TMP, puis l'évaporateur rotatif est mis sous vide afin d'éliminer le méthanol.

Le ballon tricol contenant les réactifs est ensuite placé dans un bain d'huile thermostaté à 95°C, surmonté d'une pâle d'agitation (300 rpm) sous flux d'azote. Une fois le réacteur à température, le glycidol (35.5eq, 60.03g) est ajouté avec une pompe d'ajout péristaltique à raison de 3,6mL/h.

Une fois l'ajout de glycidol terminé, le milieu réactionnel est laissé sous agitation deux heures à 95°C pour s'assurer de la conversion complète du glycidol. Le polymère obtenu est dissout dans du méthanol, neutralisé et dé-ionisé avec de l'Amberlite^{®} puis précipité deux fois dans l'acétone.

Le polyglycérol hyperramifié obtenu est caractérisé par chromatographie d'exclusion stérique (SEC) et spectroscopie RMN ¹H.

### δ (ppm), MeOD : 4.92 (OH); 3.56 (CH₂-CH₂-O)ₙ₋₂; 1.36 (CH₂, TMP); 0.87 (CH₃, TMP)

### Exemple B : Synthèse de polyglycérol hyperramifié de Mₙ= 2440 g.mol⁻¹

Le même protocole que l'exemple A a été utilisé pour produire un polyglycérol hyperramifié de masse molaire moyenne 2440 g.mol⁻¹ en changeant les proportions des réactifs selon le tableau 1.

### Exemple C : Synthèse de polyglycérol hyperramifié de Mₙ= 4037 g.mol⁻¹

Le même protocole que l'exemple 1 a été utilisé pour produire un polyglycérol de masse molaire moyenne 4037 g.mol⁻¹ en changeant les proportions des réactifs selon le tableau 1.

### Exemple D : Synthèse de macropolyol de Mₙ= 4550 g.mol⁻¹

Le même protocole que l'exemple 1 a été utilisé pour produire un polyglycérol de masse molaire moyenne 4550 g.mol⁻¹ en changeant les proportions des réactifs selon le tableau 1.

**Tableau 1 : Conditions de synthèse des polyglycérols hyperramifiés**

| **Exemples polyglycérols** | **Mn théorique (g/mol)** | **Mn exp (g/mol)** | **Masse TMP (g)** | **Masse MeOK (g)** | **Masse Glycidol (g)** |
|---|---|---|---|---|---|
| **A** | 2487 | 2760 | 3.417 | 2.126 | 60.03 |
| **B** | 2440 | 2945 | 2.9 | 1.79 | 49.98 |
| **C** | 4037 | 4200 | 5.21 | 3.65 | 151.61 |
| **D** | 4550 | 5990 | 1.523 | 3.22 | 50.08 |

### Exemple 1 : Fonctionnalisation d'un polyglycérol hyperramifié (HPG) par l'anhydride maléique, pour l'obtention d'une fonctionnalisation f=9 par macromolécule de HPG

Le polyglycérol hyperramifié (HPG de l'exemple A : 2,128g) préalablement purifié et séché sous vide est introduit dans un ballon bicol de 125mL.

Le ballon bicol contenant le réactif est ensuite placé dans un bain d'huile thermostaté à 80°C, surmonté d'une pâle d'agitation (300 rpm) sous flux d'azote. Une fois le réacteur à température, l'anhydride maléique (0,584g) est ajouté en une seule fois.

Le suivi de la réaction est effectué par spectroscopie RMN ¹H et la réaction est arrêtée lorsqu'il n'y a plus d'anhydride maléique présent dans le milieu réactionnel.

### Exemples 2 et 3 : Le même protocole est utilisé pour obtenir des fonctionnalités différentes en changeant les proportions de réactifs selon le tableau 2

**Tableau 2 : Conditions de fonctionnalisation du polyglycérol hyperramifié (HPG) par l'anhydride maléique**

| **Exempl e** | **N° HPG** | **m HPG (g)** | **m _{anhydride maléique} (g)** | **Fonctionnalité f** | **% OH fonctionnalisés** |
|---|---|---|---|---|---|
| **1** | A | 2,128 | 0,584 | 9 | 22 |
| **2** | D | 2,092 | 0,550 | 14,5 | 18 |
| **3** | C | 2,1 | 0,710 | 14,5 | 25 |

avec f : fonctionnalité d'une molécule, i.e. nombre moyen de fonctions réactives par (macro)molécule

### Exemple 4: Fonctionnalisation d'un polyglycérol (HPG) par l'anhydride itaconique, pour l'obtention d'une fonctionnalisation f=2,5, par macromolécule de HPG

Dans un ballon bicol de 125mL, le polyglycérol préalablement purifié et séché sous vide est introduit (HPG de l'exemple B : 2,046g)

Le ballon bicol contenant le réactif est ensuite placé dans un bain d'huile thermostaté à 110°C, surmonté d'une pale d'agitation (300 rpm) sous flux d'azote. Une fois le réacteur à température, l'anhydride itaconique (0,291g) est ajouté en une seule fois.

Le suivi de la réaction est effectué par spectroscopie RMN ¹H et la réaction est arrêtée lorsqu'il n'y a plus d'anhydride itaconique présent dans le milieu réactionnel.

Une fois la réaction terminée, le composé est dialysé 3 fois 4h dans du méthanol, qui est ensuite évaporé sous vide à température ambiante.

Le polymère sec est caractérisé par spectroscopie RMN ¹H.

### Exemple 5 à 11, 16 et 17 : Le même protocole est utilisé pour obtenir des fonctionnalités différentes en changeant les proportions de réactif selon le tableau 3

**Tableau 3 : Conditions de fonctionnalisation du HPG par l'anhydride itaconique**

| **Exemple** | **N° HPG** | **m HPG (g)** | **m _{anhydride itaconique} (g)** | **Fonctionnant é f** | **% OH fonctionnalisé s** |
|---|---|---|---|---|---|
| **4** | B | 2,046 | 0,291 | 2,5 | 6 |
| **5** | | | | 2,5 | 6 |
| **6** | D | 2,224 | 0.212 | 3,3 | 4 |
| **7** | A | 2,177 | 0,649 | 5,6 | 15 |
| **8** | A | 2,035 | 0,295 | 2 | 5 |
| **9** | A | 2,465 | 1,206 | 10,4 | 27 |
| **10** | A | 1,943 | 0,282 | 2 | 5 |
| **11** | B | 1,95 | 0,188 | 1,7 | 4 |
| **16** | B | 2,4 | 0,179 | 2 | 4 |
| **17** | | 2,4 | 0,179 | | |

### Exemple 12 : Fonctionnalisation d'un polyglycérol (HPG) par l'isocyanatoéthylacrylate, pour l'obtention d'une fonctionnalisation f=2, par macromolécule de HPG

Dans un ballon bicol de 125mL, le polyglycérol préalablement purifié et séché sous vide est introduit (HPG de l'exemple A : 2,046g).

Le ballon bicol contenant le réactif est ensuite placé à température ambiante, surmonté d'une pâle d'agitation (300 rpm) sous flux d'azote. Le catalyseur, dibutyle laurate d'étain, (0,018g) est ajouté avec 1,5mL de DMF préalablement distillé.

Une fois le mélange homogène l'isocyanatoéthylacrylate est ajouté (0,271g)

Le suivi de la réaction est effectué par spectroscopie RMN ¹H et la réaction est arrêtée lorsqu'il n'y a plus d'isocyanoéthylacrylate présent dans le milieu réactionnel.

### Exemple 13 et 14 : Le même protocole est utilisé pour obtenir des fonctionnalités différentes en changeant les proportions de réactif selon le tableau 4

**Tableau 4 : Conditions de fonctionnalisation du HPG par l'isocyanatoéthylacrylate**

| **Exemples** | **N° HPG** | **m HPG (g)** | **m _{isocyanatoéthylacrylate} (g)** | **f** | **% OH fonctionnalisés** |
|---|---|---|---|---|---|
| **12** | A | 2,401 | 0,271 | 2 | 5 |
| **13** | A | 2,424 | 0,986 | 7 | 18 |
| **14** | C | 2,118 | 0,263 | 3,7 | 6 |

### Exemple A1 : Procédé de préparation de gels en masse à base de polyglycérol fonctionnalisé anhydride maléique et de 2-hydroxyéthylméthacrylate

Le polyglycérol fonctionnalisé anhydride maléique (réticulant) de l'exemple 1 (0,363g, 36,3% massique) est pesé dans un flacon, puis est introduit le 2-Hydroxyéthylméthacrylate (0,632g, 63,2% massique) et l'amorceur thermique : AIBN (0,005g, 0,5% massique). Le mélange est homogénéisé au mélangeur Speedmixer pendant 30 s à 2500 rpm. Une fois homogénéisé, 0,2 mL du mélange est introduit dans un moule en polypropylène à l'aide d'une micropipette. Le moule est refermé puis placé 2h dans une étuve préalablement chauffée à 80°C. Au bout de 2h, le matériau est démoulé à chaud avant d'être caractérisé.

### Exemple A2 et A3 : Le même protocole est utilisé pour obtenir des formulations avec des pourcentages massiques de polyglycérol fonctionnalité maléique et d'HEMA, différents, selon le tableau 5

**Tableau 5 : Formulations de gels à base d'HEMA et de polyglycérol fonctionnalisé anhydride maléique.**

| **Exem pie** | **N° HPG fonctionnali sé** | **f** | **% OH fonctionnali sés** | **% massique HPG fonctionnalisé** | **% massique HEMA** | **% massique AIBN** |
|---|---|---|---|---|---|---|
| **A1** | 1 | 9 | 22 | 36,3 | 63,2 | 0,5 |
| A2 | 2 | 14,5 | 18 | 8,3 | 81,2 | 0,5 |
| A3 | 3 | 14,5 | 18 | 22 | 77,5 | 0,5 |

### Exemple A4 : Procédé de préparation de gels en masse à base de polyglycérol fonctionnalisé anhydride itaconique et de 2-hydroxyéthylméthacrylate

Le polyglycérol fonctionnalisé anhydride itaconique (réticulant) de l'exemple 4 (0,281g, 28,1% massique) est pesé dans un flacon, puis est introduit le 2-Hydroxyéthylméthacrylate (0,714g, 71,4% massique) et l'amorceur thermique : AIBN (0,005g, 0,5% massique). Le mélange est homogénéisé au mélangeur speedmixer pendant 30 s à 2500 rpm. Une fois homogénéisé, 0,2 mL du mélange est introduit dans un moule en polypropylène à l'aide d'une micropipette. Le moule est refermé puis placé 2h dans une étuve préalablement chauffée à 80°C. Au bout de 2h, le matériau est démoulé à chaud avant d'être caractérisé.

### Exemple A5 à A11, A16 : Le même protocole est utilisé pour obtenir des formulations avec des pourcentages massiques de polyglycérol fonctionnalisé anhydride itaconique et d'HEMA, différents, selon le tableau 6

### Exemple A17 : Procédé de préparation de gels en masse à base de polyglycérol fonctionnalisé anhydride itaconique, de 2-hydroxyéthylméthacrylate et d'acide méthacrylique

Le polyglycérol fonctionnalisé anhydride itaconique (réticulant) de l'exemple 4 (0,100g, 10% massique) est pesé dans un flacon, puis est introduit le 2-Hydroxyéthylméthacrylate (0,875g, 85,7% massique), l'acide méthacrylique (co-monomère) (0,02g, 2%) et l'amorceur thermique : AIBN (0,005g, 0,5% massique). Le mélange est homogénéisé au mélangeur Speedmixer pendant 30 s à 2500 rpm. Une fois homogénéisé, 0,2 mL du mélange est introduit dans un moule en polypropylène à l'aide d'une micropipette. Le moule est refermé puis placé 2h dans une étuve préalablement chauffée à 80°C. Au bout de 2h, le matériau est démoulé à chaud avant d'être caractérisé.

**Tableau 6 : Formulations de gels à base d'HEMA et de polyglycérol fonctionnalisé par de l'anhydride itaconique.**

| **exem ple** | **N° HPG fonctionn alisé** | **f** | **% OH fonctionna lisés** | **% massique HPG fonctionnal isé** | **% massi queHE MA** | **% massiqu e AIBN** | **% massique acide méthacryli que** |
|---|---|---|---|---|---|---|---|
| **A4** | 4 | 2,5 | 6 | 28,1 | 71,4 | 0,5 | / |
| **A5** | 5 | 2,5 | 6 | 4,8 | 94,7 | 0,5 | / |
| **A6** | 6 | 3,3 | 4 | 17,6 | 81,9 | 0,5 | / |
| **A7** | 7 | 5,6 | 15 | 5 | 94,5 | 0,5 | / |
| **A8** | 8 | 2 | 5 | 10 | 89,5 | 0,5 | / |
| A9 | 9 | 10,4 | 27 | 10 | 89,5 | 0,5 | / |
| A10 | 10 | 2 | 5 | 20 | 79,5 | 0,5 | / |
| **A11** | 11 | 1,7 | 4 | 11,3 | 88,2 | 0,5 | / |
| **A16** | 16 | 2 | 4 | 10 | 89,5 | 0,5 | / |
| **A17** | 17 | 2 | 4 | 10 | 87,5 | 0,5 | 2 |

### Exemple A12 : Procédé de préparation de gels en masse à base de polyglycérol fonctionnalisé isocyanatoéthylacrylate et de 2-hydroxyéthylméthacrylate

Le polyglycérol fonctionnalisé isocyanatoéthylacrylate (réticulant) de l'exemple 12 (0,091g, 9,1% massique) est pesé dans un flacon, puis est introduit le 2-Hydroxyéthylméthacrylate (0,904g, 90,4% massique) et l'amorceur thermique : AIBN (0,005g, 0,5% massique). Le mélange est homogénéisé au mélangeur speedmixer pendant 30 s à 2500 rpm. Une fois homogénéisé, 0,2 mL de mélange est introduit dans un moule en polypropylène à l'aide d'une micropipette. Le moule est refermé puis placé 2h dans une étuve préalablement chauffée à 80°C. Au bout de 2h, le matériau est démoulé à chaud avant d'être caractérisé.

### Exemple A13 et A14 : Le même protocole est utilisé pour obtenir des formulations avec des pourcentages massiques de polyglycérol fonctionnalisé isocyanatoéthylacrylate et d'HEMA, différents, selon le tableau 7

**Tableau 7 : Différentes formulations de gels contenant du polyglycérol fonctionnalisé isocyanatoéthylacrylate et de l'HEMA.**

| **Exemple** | **N° HPG fonctionnalisé** | **f** | **% OH fonctionnalisés** | **% massique HPG fonctionnalisé** | **% massique HEMA** | **% massique AIBN** |
|---|---|---|---|---|---|---|
| **A12** | 12 | 2 | 5 | 9,1 | 90,4 | 0,5 |
| **A13** | 13 | 7 | 18 | 4,5 | 95 | 0,5 |
| **A14** | 14 | 3,7 | 6 | 4,8 | 94,7 | 0,5 |

### Exemple A15 : Procédé de préparation de gels en masse à base de 2-hydroxyéthylméthacrylate et de triéthylène glycol diméthylméthacrylate (TEGDMA), utilisé comme référence

Le 2-Hydroxyéthylméthacrylate (0,990g, 99% massique) est pesé dans un flacon, puis sont introduits le TEGDMA (0,005g, 0,5% massique) et l'amorceur thermique : AIBN (0,005g, 0,5% massique). Le mélange est homogénéisé au mélangeur speedmixer pendant 30 s à 2500 rpm. Une fois homogénéisé, 0,2 mL de mélange est introduit dans un moule en polypropylène à l'aide d'une micropipette. Le moule est refermé puis placé 2h dans une étuve préalablement chauffée à 80°C. Au bout de 2h, le matériau est démoulé à chaud avant d'être caractérisé.

### Exemple A18: Le même protocole est utilisé pour obtenir des formulations avec des pourcentages massiques de TEGDMA et d'HEMA, différents, selon le tableau 8

**Tableau 8 : Différentes formulations de gels contenant de l'HEMA et du TEGDMA**

| **exemple** | **f** | **% massique de TEGDMA** | **% massique HEMA** | **% massique AIBN** |
|---|---|---|---|---|
| **A15** | 2 | 0,5 | 99 | 0,5 |
| A18 | 2 | 0,5 | 99 | 0,5 |

**Tableau 9 : Teneurs en eau des hydrogels (EWC en %) déterminées dans l'eau distillée et en solution tampon phosphate (pH=7,4, osmolarité = 280 mOsm)**

| **Exemple** | **EWC (eau)** | **EWC (tampon phosphate)** |
|---|---|---|
| **A1** | 71 | / |
| **A2** | 53 | / |
| **A3** | 44 | / |
| **A4** | 61 | / |
| **A5** | 52 | / |
| **A6** | 57 | / |
| **A7** | 43 | 44 |
| **A8** | 50 | 50 |
| **A9** | 43 | 48 |
| **A10** | 50 | / |
| **A11** | 44 | / |
| **A12** | 49 | 46 |
| **A13** | 42 | 42 |
| **A15** | 41 | 39 |
| A18 | 40 | 41 |

**Tableau 10 : Evolution à l'air ambiant de la teneur en eau (EWC en %) en fonction du temps d'exposition des gels gonflés à l'eau distillée (Dimensions des hydro gels testés : diamètre 23 mm ; épaisseur 500 µm)**

| | **Rétention dans eau distillée (EWC)** | | | |
|---|---|---|---|---|
| **exemple** | **0 min** | **30 min** | **76 min** | **131 min** |
| **A1** | 71 | 69 | 65 | 57 |
| **A4** | 61 | 56 | 44 | 31 |
| **A15** | 42 | 38 | 31 | 25 |
| A18 | 41 | 33 | 27 | 14 |

| **exemple** | **0 min** | **15 min** | **30 min** | **60 min** |
|---|---|---|---|---|
| **A16** | 56 | 52 | 48 | 29 |
| **A17** | 52 | 51 | 46 | 32 |
| A18 | 41 | 36 | 33 | 29 |

**Tableau 11 : Evolution à l'air ambiant de la teneur en eau (EWC en %) en fonction du temps d'exposition des gels gonflés par une solution tampon phosphate (Dimensions des hydro gels testés : diamètre 23mm ; épaisseur 500 µm)**

| | **Rétention dans solution tampon (EWC)** | | | |
|---|---|---|---|---|
| **Exemples** | **0 min** | **15 min** | **30 min** | **60 min** |
| **A16** | 51 | 49 | 44 | 28 |
| **A17** | 59 | 56 | 52 | 39 |
| A18 | 40 | 36 | 32 | 15 |

**Tableau 12 : Temps nécessaires pour atteindre une perte en eau de 10% massique (T₁₀) et 50% massique (T₅₀)**

| **Exemples** | **T₁₀ (min)** | **T₅₀ (min)** |
|---|---|---|
| **A1** | 82 | 251 |
| **A4** | 30 | 128 |
| A18 | 20 | 98 |

**Tableau 13 : Propriétés mécaniques de différentes formulations**

| **Exemples** | **Module (MPa)** | **Contrainte à la Rupture(MPa)** | **Elongation à la rupture (%)** |
|---|---|---|---|
| **A3** | 0,558 | 1,035 | 240 |
| **A6** | 0,893 | 0,608 | 269 |
| **A7** | 0,58 | 0,788 | 319 |
| **A10** | 0,566 | 0,558 | 314 |
| **A12** | 0,94 | 1,475 | 358 |
| **A13** | 1,11 | 0,769 | 164 |
| **A15** | 0,772 | 0,798 | 333 |

**Tableau 14 : Perméabilité à l'oxygène**

| **Exemples** | **Dk (barrer)** |
|---|---|
| **A3** | 21.3 |
| **A11** | 12,3 |
| **A13** | 12,4 |
| **A15** | 15,8 |

### Liste des références

1.US20010005738.
2. WO2017150786.
3. Frey et al., Acta Polym. 1997,48, 30.
4. Journal of The American Society (2000) 122, 2954-2955.
5. Sunder et al. : « Controlled Synthesis of Hyperbranched Polyglycerols by Ring-Opening Multibranching Polymerization », Macromolecules 1999, 32, 13, 4240-4246.
6. Kainthan et al. : « Synthesis, Characterization, and Viscoelastic Properties of High Molecular Weight Hyperbranched Polyglycerols », Macromolecules 2006, 39, 22, 7708-7717.

## Revendications

1. Composition réticulable comprenant :
A) au moins un monomère polymérisable ou un mélange de monomères polymérisables ou un mélange de monomères et de macromonomères polymérisables portant au moins une fonction C=C polymérisable de type acrylate, méthacrylate, maléate, fumarate, itaconate, citraconate, styrénique ou vinylique ;
B) au moins un polyol choisi parmi les oligoglycérols, les polyglycérols linéaires, ramifiés et hyperramifiés, ou un mélange de polyglycérol hyperramifié et d'un polyol choisi parmi les oligoglycérols, et les polyglycérols linéaires, ramifiés et hyperramifiés, comprenant en moyenne plus de 1 groupement hydroxyle fonctionnalisé avec un groupement portant une fonction C=C polymérisable de type isocyanatoalkyl(méth)acrylate, maléate, fumarate, itaconate, citraconate, styrénique ou vinylique ;
C) un amorceur radicalaire ou un mélange d'amorceurs radicalaires pouvant amorcer la polymérisation par voies thermiques, rédox ou photochimiques; et
D) éventuellement au moins un additif choisi parmi les antioxydants, les agents d'ajustement des propriétés physico-chimiques, les agents de promotion, de modulation et / ou de contrôle de la perméabilité à l'oxygène, les plastifiants, les agents humectants, les lubrifiants, les agents de modification de la viscosité, les agents compatibilisants, les agents de coloration, les agents filtrants, les agents anti-microbiens, les agents thérapeutiques et les anti-biofilms bactériens.

2. Composition réticulée susceptible de former un polymère hydrogel, issue de la réticulation de:
A) au moins un monomère polymérisable ou mélange de monomères polymérisables ou un mélange de monomères et de macromonomères polymérisables portant au moins une fonction C=C polymérisable de type acrylate, méthacrylate, maléate, fumarate, itaconate, citraconate, styrénique ou vinylique ;
B) au moins un polyol choisi parmi les oligoglycérols, les polyglycérols linéaires, ramifiés et hyperramifiés ou un mélange de polyglycérol hyperramifié et d'un polyol choisi parmi les oligoglycérols et les polyglycérols linéaires, ramifiés et hyperramifiés, comprenant en moyenne plus de 1 groupement hydroxyle fonctionnalisé avec un groupement portant une fonction C=C polymérisable de type isocyanatoalkyl(méth)acrylate, maléate, fumarate, itaconate, citraconate, styrénique ou vinylique ;
C) un amorceur radicalaire ou un mélange d'amorceurs radicalaires susceptible d'amorcer la polymérisation par voies thermiques, rédox ou photochimiques; et
D) éventuellement au moins un additif choisi parmi les antioxydants, les agents permettant d'ajuster les propriétés physico-chimiques telles que le module d'Young, l'élongation et la contrainte à la rupture, les agents de promotion, de modulation et /ou de contrôle de la perméabilité à l'oxygène, les plastifiants, les agents humectants, les lubrifiants, les réducteurs de viscosité, les agents compatibilisants, les agents de coloration, les agents filtrants, les agents anti-microbiens, les agents thérapeutiques et les anti-biofilms bactériens.

3. Composition selon l'une quelconque des revendications 1 à 2, dans laquelle le polyglycérol hyperramifié fonctionnalisé, est issu d'un polyglycérol hyperramifié ayant une masse molaire moyenne en nombre comprise entre 546 et 100 000 g/mol, et présente un degré de ramification (DB) compris entre 0,3 et 1,0, déterminés selon les méthodes mentionnées dans la description.

4. Composition selon l'une quelconque des revendications 1 ou 2, dans laquelle le polyglycérol hyperramifié est un dendrimère de polyglycérol ou un polyglycérol hyperramifié de degré de ramification compris entre 0,3 et 1,0, déterminé selon la méthode mentionnée dans la description, ou un mélange de polyglycérol hyperramifié et d'oligoglycérols ou de polyglycérols linéraires.

5. Composition selon l'une quelconque des revendications 1 à 4, dans laquelle la fonction C=C polymérisable sur le polyglycérol hyperramifié fonctionnalisé, est choisie parmi les groupements isocyanatoalkyl(méth)acrylates, maléiques, fumariques, itaconiques, citraconique ou vinyliques de structure: 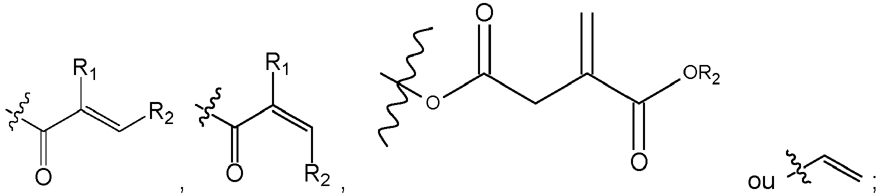 dans laquelle R₁ représente H ou méthyle ; R₂ représente H, C₁₋₆alkyle linéaire ou ramifié, ou -C(=O)OR_{2A} dans lequel R_{2A} représente H ou C₁₋₆alkyle linéaire ou ramifié éventuellement substitué par un ou plusieurs groupements hydroxyles, et R₃ représente H, C1-20 alkyle linéaire ou ramifié.

6. Composition selon la revendication 1 à 5, dans laquelle le isocyanatoalkyl(méth)acrylate est choisi parmi le 2-isocyanatoéthylméthacrylate et le 2-isocyanatoéthyl acrylate ou leur mélange.

7. Composition selon l'une quelconque des revendications 1 à 6, dans laquelle dans le constituant A) est choisi parmi :
- l'acide acrylique ou l'acide méthacrylique ainsi que les alkyl (méth)acrylates et leurs dérivés, tels que le 2-hydroxyéthylméthacrylate (HEMA), le 2-hydroxyéthylacrylate (HEA), le méthylméthacrylate (MMA), le méthacrylamide, le N,N-diméthyl-diacétoneacrylamide, le 2-phosphatoéthylméthacrylate, les mono-, di-, tri-, tétra-, penta-polyéthylèneglycol acrylates ou méthacrylates, la N-(3-méthacrylamidopropyl)-N,N-diméthylamine, la N-(3-méthacrylamidopropyl)-N,N,N-triméthylamine, la N-(3-acrylamido-3-méthylbutyl)-N,N-diméthylamine, le N-méthylacrylamide, le 3-hydroxypropyl méthacrylate, le propyl méthacrylate, le propyl acrylate, le butyl méthacrylate, le butyl acrylate, le pentyl acrylate, le pentyl méthacrylate, le N-(1,1-diméthyl-3-oxobutyl)acrylamide, le 2-éthyl-2-(hydroxy-méthyl)-1,3-propanediol le triméthacrylate, le butyl(méth)acrylate, le 2-hydroxybutyl méthacrylate, le 3-hydroxy-2-naphthyl méthacrylate, le N-(formylméthyl)acrylamide, le 2-éthoxyéthyl méthacrylate, le 4-t-butyl-2-hydroxycyclohexyl méthacrylate, ou les isocyanatoalkyl(méth)acrylates comme le 2-isocyanatoéthylméthacrylate, le 2-Isocyanatoéthyl acrylate, le 4 - [(a) -phényl diazényl] phényl-2-méthacrylate (4 - [(E) - phényldiazényl] phényl-2-méthacrylate) ou le 4 - [(a) -phényl diazényl] phényl-2-méthacrylate (4 - [(E) -phényldiazényl] phényl-2-méthacrylate),
- les monomères maléates et fumarates, tels que l'acide maléique, l'anhydride maléique, le diméthylmaléate, le diéthylmaléate, ou les esters de maléate supérieurs dans lesquels la chaîne alkyle contient 3 à 20 atomes de carbone ainsi que l'acide fumarique, le diméthylfumarate, le diéthylfumarate ou les esters de fumarate supérieurs dans lesquels la chaîne alkyle contient 3 à 20 atomes de carbone,
- les monomères itaconates ; tels que l'acide itaconique, l'anhydride itaconique, le diméthylitaconate, ou les esters d'itaconate supérieurs dans lesquels la chaîne alkyle contient 3 à 20 atomes de carbone,
- les monomères citraconates ; tels que l'acide citraconique, l'anhydride citraconique, le diméthylcitraconate, ou les esters de citraconate supérieurs dans lesquels la chaîne alkyle contient 3 à 20 atomes de carbone,
- les monomères (méth)acryliques pouvant contenir des fonctions silicones ou silanes ; par exemple le méthacrylate de tris(triméthylsiloxy)silylpropyle, le méthacrylate de bis(triméthylsiloxy)méthylsilylpropyle, le méthacrylate de pentaméthyldisiloxanepropyle, le méthacrylate de tris(triméthylsiloxy) silylpropyloxyéthyle, le méthylacryloxyéthylcarbamate de tris(triméthylsiloxy)-silylpropyle, le méthacrylate de tris(triméthylsiloxy)silylpropylglycérol, l'acrylate de phényltétraméthyle-disiloxanyléthyle, le 3-méthacryloxypropylbis(triméthylsiloxy)méthylsilane, le méthyl-di(triméthylsiloxy)méthacryloxyméthyle silane, le (3-méthacryloxy-2-hydroxypropyloxy) propyle bis (triméthylsiloxy)méthylsilane, le 3-acryloxypropyl tris(triméthylsiloxy)Silane, le 3-acryloxypropyltrichlorosilane, 3-méthacryloxypropyl diméthyl Ethoxysilane, 3-méthacryloxypropyl méthyl diéthoxysilane, le méthacryloxypropylbis(triméthylsiloxy)méthylsilane, le tristriméthylsilyloxysilylpropyl méthacrylate, le bis(méthacryloxypropyle) tétraméthyldisiloxane, l'acryloxyméthyltriméthylsilane, le p-(t-butyldiméthylsiloxy)styrène, le 1,3-bis(3-méthacryloxypropyl)tétrakis(triméthylsiloxy)disiloxane, le 3 triméthylsilylpropargylméthacrylate, l'acrylate de 3-(triméthoxysilyl)propyle,
- les monomères vinyliques pouvant contenir des fonctions silicones ou silanes ; par exemple le vinyl(chlorométhyl)diméthoxysilane, le vinyléthoxysiloxane-propyléthoxysiloxane, le vinyltris(1-méthoxy-2-propoxy)silane, le vinyltris(2-méthoxyéthoxy)silane, le vinyltriméthoxysilane, le vinyltriisopropoxysilane, le vinyltriéthoxysilane, le vinyltrichlorosilane, le vinyl tri-t-butoxysilane, le o-(vinyloxybutyl)-n-triéthoxysilylpropyl carbamate, le vinylméthyldiméthoxysilane, le vinylméthyldiéthoxysilane, le vinyldiméthyléthoxysilane, le 3-[tris (triméthylsiloxy)silyle] propyl vinyl carbamate, l'acide triéthoxysilylpropyl maléique ou le 1,3,5-trivinyl-1,1,3,5,5-pentaméthyltrisiloxane,
- les monomères fluorés acryliques et méthacryliques, tels que le dihydroperfluorooctyle acrylate, le 1,1-dihydroperfluorobutyle acrylate, le trihydroperfluoroalkyle acrylate, le tétrahydroperfluoroalkylacrylate, le tris(triméthylsilyloxy)propyle méthacrylate, le perfluoro hexyléthylthiocarbonylaminoéthyle méthacrylate, le trifluoroéthyle méthacrylate, l'hexafluoroisopropyle méthacrylate, l'hexafluorobutyle méthacrylate, le trifluoroéthyle méthacrylate,
- les composés prépolymères organosilanes, tels que le α,ω-bisméthacryloxy-propyle polydiméthylsiloxane, les polydiméthylsiloxanes à terminaison(s) vinylique(s), les copolymères diphénylsiloxane-diméthylsiloxane à terminaisons vinyliques, les copolymères trifluoropropylméthylsiloxane à terminaison(s) vinylique(s), les copolymères diéthylsiloxane-diméthylsiloxane à terminaison(s) vinylique(s), les copolymères d'éthylsiloxane diméthylsiloxane à terminaison(s) vinylique(s), les copolymères éthylène-siloxane à terminaison(s) vinylique(s), le méthacrylate de tris(polydiméthylsiloxy)silylpropyle, les (méth)acrylates de polysiloxanylalkyle, les méthacryloxypropyle à terminaison polydiméthylsiloxane, le 1,3-divinyl-1,1,3,3-tétraméthyldisiloxane, le 2-(divinylméthylsilyl)éthyltriéthoxysilane, ou le 1,3-divinyl-1,3-diphényl-1,3-diméthyldisilane,
- les composés mixtes fluorés / silanes ou chlorés / silanes, tels que le fluoro-méthacryloxypropyl tris(triméthylsiloxy) silane, 3-acryloxy propyl Méthyl dichlorosilane, le 3-acryloxypropyltrichlorosilane, le 3-méthacryloxypropyl trichlorosilane,
- les composés silanes / isocyanates tel que le 3-Isocyanatopropyltriéthoxysilane, et
- les monomères vinyliques, tels que les N-vinyl lactames ; par exemple N-vinyl pyrrolidone (NVP)), N-vinyl-N-methyl acétamide (VMA), N-vinyl-N-éthyl acétamide, N-vinyl-N-ethyl formamide, N-vinyl formamide, N-2-hydroxyéthyl vinyl carbamate, N-carboxy-alanine N-vinyl ester, de préférence CH₃C(O)N(CH₃)-CH=CH₂ (N-vinyl-N-methyl acétamide (VMA)).

8. Composition selon l'une quelconque des revendications 1 à 7, dans laquelle l'amorceur est au moins un amorceur UV, thermique et/ou redox, choisi parmi :
- un amorceur photochimique choisi dans le groupe comprenant la 2-hydroxy-4-acryloyloxyéthoxy benzophénone, la 2-hydroxy-4-(2-hydroxy-3-méthacrylyloxy) propoxybenzophénone ; la benzophénone, l'acétophénone, l'acétonaphthoguinone, la méthyl éthyl cétone, la valérophénone, l'hexanophénone, la C-phénylbutyrophénone, la p-morpholinopropiophénone, la dibenzosubérone, la 4-morpholinobenzophénone, la 4-morpholinodéoxybenzoïne, le p-diacétylbenzène, la 4-aminobenzophénone, la para-méthoxyacétophénone, la 3-méthylanthraquinone, la tert-butylanthraquinone, les esters d'anthraquinone, le benzaldéhyde, le 9-acétylphénanthrène, le 2-acétylphénanthrène, la 10-thioxanthénone, le 3-acétylphénanthrène, le 3-acétylindole, la 9-fluorénone, la 1-indanone, le 1,3,4-triacétylbenzène, la thioxanthèn-9-one, la xanthèn-9-one, la 2,4-diméthylthioxanthone,la 2,4-diéthylthioxanthone, la 2,4-di-iso-propylthioxanthone, la 2,4-dichlorothioxanthone, la benzoïne, la benzoïne isobutyléther, la benzoïne tétrahydropyranyléther, la benzoïne méthyléther, la benzoïne éthyléther, la benzoïne butyléther, la benzoïne isopropyléther, la chloroxanthenone, la 7-H- benzoïne méthyl éther, la benzodéanthracèn-7-one, le 1-naphthaldéhyde, la 4,4'-bis(dimethylamino)benzophénone, la 4-phénylbenzophénone, la 4-chlorobenzophénone, la 4,4'-bis(diméthylamino)benzophénone, la 1-acétonaphthone, la 2-acétonaphthone, le 1-benzoylcyclohexan-1-ol, la 2-hydroxy-2,2-diméthylacétophénone, la 2'2-diméthoxy-2-phénylacétophénone, la 2,2-diéthoxy-2-phénylacétophénone, la 1,1-dichloroacétophénone, la 1-hydroxyacétophénone,l'acétophénone diméthyl acétal, l'o-méthoxybenzophénone,la triphénylphosphine, la tris-o-tolylphosphine, la benzaanthracène-7,12-dione, la 2,2-diéthoxyacétophénone, les benzyl acétals tels que le benzyl diméthyl acétal, la 2-méthyl-1-4-(méthylthio)phényl-2-morpholinopropan-1-one, les anthraquinones telles que la 2-méthylanthraquinone, la 2-éthylanthraquinone, la 2-tert-butylanthraquinone, la 1- chloroanthraquinone, la 2-amylanthraquinone, la 2,3-butanedione,
- un photo-amorceur choisi dans le groupe comprenant les esters de l'acide phénylglyoxalique, et leurs combinaisons entre eux ou avec des promoteurs de photopolymérisation tels que l'acide benzoïque ou des amines,
- un amorceur thermique choisi dans le groupe comprenant le 2,2-azobis(2,4-diméthylpentanenitrile), le 2,2'-azobisisobutyronitrile, le 1,1'-azobis(cyclohexanecarbonitrile), 2,2'-azobis(2-méthylpropionitrile), le péroxodisulfate de potassium, le péroxyde de dibenzoyle, le péroxyde de cyclohexanone, le péroxyde di-tert-butyle, le péroxyde d'acétyle cyclohexylsulfonyle, le percarbonate de diisopropyle, péroctoate ou benzpinacol de tert-butyle, le péroxyde de di-t-butyle, l'hydropéroxyde de cumène, le péroxyde de dicumyle, le perbenzoate de t-butyle, et l'amine N-oxyde hydroxylée, comme le 2,2,6,6-tétraméthylpipéridine-N-oxyl et le 4-hydroxy-2,2,6,6-tétraméthylpipéridine-N-oxyl,
- un système d'amorceur rédox choisi dans le groupe comprenant des oxydants tels que l'AIBN, les péroxydes comme le péroxyde d'hydrogène, le péroxyde de benzoyle, le t-butyl-hydropéroxyde, l'hydropéroxyde de p-mentane, le persulfate d'ammonium, le persulfate de sodium, le persulfate de potassium, des réducteurs tels que l'acide ascorbique, le bisulfite de sodium, la N,N,N',N'-tétraméthyléthylène diamine le formaldéhyde sulfoxylate de sodium, les sels de métaux Fe²⁺, Cr²⁺, Zn²⁺, V²⁺, Ti³⁺, Co²⁺, Cu⁺ comme le sulfate de Fer, et les N,N-dialkylanilines tels que la N,N-diméthyl-p-toluidine.

9. Hydrogel susceptible d'être obtenu par :
- hydratation/gonflement à partir d'eau ou d'une solution aqueuse d'une composition réticulée selon l'une quelconque des revendications 2 à 8 ou l'une quelconque des revendications , ou,
- polymérisation de la composition réticulable selon la revendication 1 ou l'une quelconque des revendications 3 à 8, en présence d'un solvant protique ou aprotique miscible à l'eau, puis échange du solvant avec un excès d'eau ou une solution aqueuse, ou
- par polymérisation de la composition réticulable selon la revendication 1 ou l'une quelconque des revendications 3 à 8, en présence d'eau ou d'une solution aqueuse.

10. Procédé de préparation d'une composition réticulée telle que définie dans la revendication 2 consistant à faire réagir, en présence ou en l'absence de solvant aprotique, de préférence en l'absence de solvant :
A) au moins un monomère polymérisable ou mélange de monomères polymérisables ou un mélange de monomères et de macromonomères polymérisables portant au moins une fonction C=C polymérisable de type acrylate, méthacrylate, maléate, fumarate, itaconate, citraconate, styrénique ou vinylique
B) au moins un polyol choisi parmi les oligoglycérols et les polyglycérols linéaires, ramifiés et hyperramifié, ou un mélange de polyglycérol hyperramifié et d'un polyol choisi parmi les oligoglycérols et les polyglycérols linéaires, ramifiés et hyperramifiés, comprenant en moyenne plus de 1 groupements hydroxyles fonctionnalisés avec un groupement portant une fonction C=C polymérisable de type isocyanatoalkyl(méth)acrylate, maléate, fumarate, itaconate, citraconate, styrénique ou vinylique ;
C) un amorceur radicalaire ou un mélange d'amorceurs radicalaires pouvant amorcer la polymérisation par voies thermiques, rédox ou photochimiques; et
D) éventuellement au moins un additif choisi parmi les antioxydants, les agents permettant d'ajuster les propriétés physico-chimiques telles que le module d'Young, l'élongation et la contrainte à la rupture, les promoteurs de la perméabilité à l'oxygène, les plastifiants, les agents humectants, les lubrifiants, les agents de modification de la viscosité, les agents compatibilisants, les agents de coloration, les agents filtrants, les agents anti-microbiens, les agents thérapeutiques et les anti-biofilms bactériens.

11. Procédé selon la revendication 10, dans lequel le polyglycérol hyperramifié fonctionnalisé est présent à raison de 0,1 à 99,99% massique du poids total des constituants A) à D), ou dans lequel l'amorceur est présent à raison de 0,02 à 5% massique du poids total des constituants A) à D).

12. Procédé selon l'une quelconque des revendications 10 ou 11, comprenant en outre une étape de moulage de la composition réticulée.

13. Article susceptible d'être obtenu par un procédé selon l'une quelconque des revendications 10 à 12.

14. Article selon la revendication 13, lequel est choisi parmi une lentille de contact ou intraoculaire, un patch, un implant ou un pansement.

15. Utilisation d'une composition ou d'un hydrogel selon l'une quelconque des revendications 1 à 9 pour la fabrication d'un dispositif médical, ou comme vecteur d'un composé d'intérêt choisi parmi les principes actifs thérapeutiques, les vitamines, les nutriments, les agents décontaminants ou les produits lubrifiants, ou dans des applications biomédicales, cosmétiques, ou sanitaires.

## Patentansprüche

1. Vernetzbare Zusammensetzung, umfassend:
A) mindestens ein polymerisierbares Monomer oder eine Mischung von polymerisierbaren Monomeren oder eine Mischung von Monomeren und polymerisierbaren Makromonomeren, die mindestens eine polymerisierbare C=C-Funktion vom Acrylat-, Methacrylat-, Maleat-, Fumarat-, Itaconat-, Citraconat-, Styrol- oder Vinyltyp tragen;
B) mindestens ein Polyol, ausgewählt aus Oligoglycerolen, linearen, verzweigten und hyperverzweigten Polyglycerolen, oder eine Mischung von hyperverzweigtem Polyglycerol und einem Polyol, ausgewählt aus Oligoglycerolen und linearen, verzweigten und hyperverzweigten Polyglycerolen, die im Durchschnitt mehr als 1 Hydroxylgruppe umfassen, die mit einer Gruppe funktionalisiert sind, die eine polymerisierbare C=C-Funktion vom Isocyanatoalkyl(meth)acrylat-, Maleat-, Fumarat-, Itaconat-, Citraconat, Styrol- oder Vinyltyp trägt;
C) einen Radikalinitiator oder eine Mischung von Radikalinitiatoren, die die Polymerisation auf thermischen, Redox- oder photochemischen Wegen initiieren; und
D) gegebenenfalls mindestens einen Zusatzstoff, ausgewählt aus Antioxidationsmitteln, Mitteln zur Anpassung der physiko-chemischen Eigenschaften, Mitteln zur Förderung, Modulation und/oder Steuerung der Sauerstoffdurchlässigkeit, Weichmachern, Feuchthaltemitteln, Schmiermitteln, Viskositätsmodifikationsmitteln, Kompatibilisierungsmitteln, Färbemitteln, Filtriermitteln, antimikrobiellen Mitteln, therapeutischen Mitteln und Mitteln gegen bakterielle Biofilme.

2. Vernetzte Zusammensetzung, die ein Hydrogelpolymer bilden kann, die aus der Vernetzung hervorgeht von:
A) mindestens einem polymerisierbaren Monomer oder einer Mischung von polymerisierbaren Monomeren oder einer Mischung von polymerisierbaren Monomeren und Makromonomeren, die mindestens eine polymerisierbare C=C-Funktion vom Acrylat-, Methacrylat-, Maleat-, Fumarat-, Itaconat-, Citraconat-, Styrol- oder Vinyltyp tragen;
B) mindestens einem Polyol, ausgewählt aus Oligoglycerolen, linearen, verzweigten und hyperverzweigten Polyglycerolen oder einer Mischung von hyperverzweigtem Polyglycerol und einem Polyol, ausgewählt aus Oligoglycerolen und linearen, verzweigten und hyperverzweigten Polyglycerolen, die im Durchschnitt mehr als 1 Hydroxylgruppe umfassen, die mit einer Gruppe funktionalisiert sind, die eine polymerisierbare C=C-Funktion vom Isocyanatoalkyl(meth)acrylat-, Maleat-, Fumarat-, Itaconat-, Citraconat, Styrol- oder Vinyltyp trägt;
C) einem Radikalinitiator oder einer Mischung von Radikalinitiatoren, die die Polymerisation auf thermischen, Redox- oder photochemischen Wegen initiieren; und
D) gegebenenfalls mindestens einem Zusatzstoff, ausgewählt aus Antioxidationsmitteln, Mitteln zum Anpassen der physiko-chemischen Eigenschaften wie den Elastizitätsmodul, die Zug- und Bruchspannung, Mitteln zur Förderung, Modulation und/oder Steuerung der Sauerstoffdurchlässigkeit, Weichmachern, Feuchthaltemitteln, Schmiermitteln, Viskositätsverringerern, Kompatibilisierungsmitteln, Färbemitteln, Filtriermitteln, antimikrobiellen Mitteln, therapeutischen Mitteln und Mitteln gegen bakterielle Biofilme.

3. Zusammensetzung nach einem der Ansprüche 1 bis 2, wobei das funktionalisierte hyperverzweigte Polyglycerol aus einem hyperverzweigten Polyglycerol hervorgeht, das eine zahlenmittlere molare Masse zwischen 546 und 100.000 g/mol aufweist und einen Verzweigungsgrad (DB) zwischen 0,3 und 1,0 aufweist, bestimmt gemäß den in der Beschreibung erwähnten Verfahren.

4. Zusammensetzung nach einem der Ansprüche 1 oder 2, wobei das hyperverzweigte Polyglycerol ein Polyglyceroldendrimer oder ein hyperverzweigtes Polyglycerol mit einem Verzweigungsgrad zwischen 0,3 und 1,0, bestimmt gemäß dem in der Beschreibung erwähnten Verfahren, oder eine Mischung von hyperverzweigtem Polyglycerol und Oligoglycerolen oder linearen Polyglycerolen ist.

5. Zusammensetzung nach einem der Ansprüche 1 bis 4, wobei die polymerisierbare C=C-Funktion an dem funktionalisierten hyperverzweigten Polyglycerol ausgewählt ist aus Isocyanatoalkyl(meth)acrylat-, Maleinsäure-, Fumarsäure-, Itaconsäure-, Citraconsäure- oder Vinylgruppen von folgender Struktur: 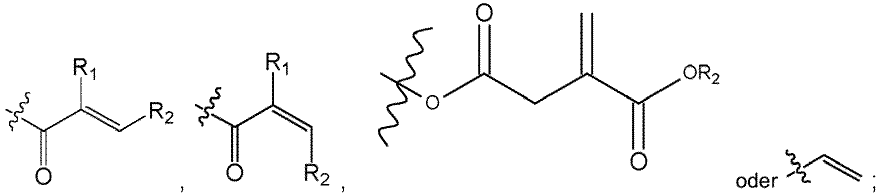 wobei R₁ H oder Methyl darstellt; R₂ H, lineares oder verzweigtes C₁₋₆-Alkyl oder -C(=O)OR_{2A} darstellt, wobei R_{2A} H oder lineares oder verzweigtes C₁₋₆-Alkyl darstellt, gegebenenfalls substituiert durch eine oder mehrere Hydroxylgruppen, und R₃ H, lineares oder verzweigtes C1-20-Alkyl darstellt.

6. Zusammensetzung nach Anspruch 1 bis 5, wobei das Isocyanatoalkyl(meth)acrylat ausgewählt ist aus 2-Isocyanatoethylmethacrylat und 2-Isocyanatoethylacrylat oder deren Mischung.

7. Zusammensetzung nach einem der Ansprüche 1 bis 6, wobei der Bestandteil A) ausgewählt ist aus:
- Acrylsäure oder Methacrylsäure sowie Alkyl(meth)acrylaten und deren Derivaten, wie 2-Hydroxyethylmethacrylat (HEMA), 2-Hydroxyethylacrylat (HEA), Methylmethacrylat (MMA), Methacrylamid, N,N-Dimethyldiacetonacrylamid, 2-Phosphatoethylmethacrylat, Mono-, Di-, Tri-, Tetra-, Pentapolyethylenglycolacrylate oder -methacrylate, N-(3-Methacrylamidopropyl)-N,N-dimethylamin, N-(3-Methacrylamidopropyl)-N,N,N-trimethylamin, N-(3-Acrylamido-3-methylbutyl)-N,N-dimethylamin, N-Methylacrylamid, 3-Hydroxypropylmethacrylat, Propylmethacrylat, Propylacrylat, Butylmethacrylat, Butylacrylat, Pentylacrylat, Pentylmethacrylat, N-(1,1-Dimethyl-3-oxobutyl)acrylamid, 2-Ethyl-2-(hydroxymethyl)-1,3-propandiol Trimethacrylat, Butyl(meth)acrylat, 2-Hydroxybutylmethacrylat, 3-Hydroxy-2-naphthylmethacrylat, N-(Formylmethyl)acrylamid, 2-Ethoxyethylmethacrylat, 4-t-Butyl-2-hydroxycyclohexylmethacrylat oder Isocyanatoalkyl(meth)acrylate wie 2-Isocyanatoethylmethacrylat, 2-Isocyanatoethylacrylat, 4-[(a)-Phenyldiazenyl]phenyl-2-methacrylat (4-[(E)-Phenyldiazenyl]phenyl-2-methacrylat) oder 4-[(a)-Phenyldiazenyl]phenyl-2-methacrylat (4-[(E)-Phenyldiazenyl]phenyl-2-methacrylat),
- Maleat- und Fumaratmonomeren, wie Maleinsäure, Maleinsäureanhydrid, Dimethylmaleat, Diethylmaleat oder höhere Maleatester, in denen die Alkylkette 3 bis 20 Kohlenstoffatome enthält, sowie Fumarsäure, Dimethylfumarat, Diethylfumarat oder höhere Fumaratester, in denen die Alkylkette 3 bis 20 Kohlenstoffatome enthält,
- Itaconatmonomeren; wie Itaconsäure, Itaconsäureanhydrid, Dimethylitaconat oder höhere Itaconatester, in denen die Alkylkette 3 bis 20 Kohlenstoffatome enthält,
- Citraconatmonomeren; wie Citraconsäure, Citraconsäureanhydrid, Dimethylcitraconat oder höhere Citraconatester, in denen die Alkylkette 3 bis 20 Kohlenstoffatome enthält,
- (Meth)acrylsäuremonomeren, die Silikon- oder Silanfunktionen enthalten können; zum Beispiel Tris(trimethylsiloxy)silylpropylmethacrylat, Bis(trimethylsiloxy)methylsilylpropylmethacrylat, Pentamethyldisiloxanpropylmethacrylat, Tris(trimethylsiloxy)silylpropyloxyethylmethacrylat, Tris(trimethylsiloxy)silylpropylmethylacryloxyethylcarbamat, Tris(trimethylsiloxy)silylpropylglycerolmethacrylat, Phenyltetramethyldisiloxanylethylacrylat, 3-Methacryloxypropylbis(trimethylsiloxy)methylsilan, Methyldi(trimethylsiloxy)methacryloxymethylsilan, (3-Methacryloxy-2-hydroxypropyloxy)propylbis(trimethylsiloxy)methylsilan, 3-Acryloxypropyltris(trimethylsiloxy)silan, 3-Acryloxypropyltrichlorsilan, 3-Methacryloxypropyldimethylethoxysilan, 3-Methacryloxypropylmethyldiethoxysilan, Methacryloxypropylbis(trimethylsiloxy)methylsilan, Tristrimethylsilyloxysilylpropylmethacrylat, Bis(methacryloxypropyl)tetramethyldisiloxan, Acryloxymethyltrimethylsilan, p-(t-Butyldimethylsiloxy)styrol, 1,3-Bis(3-methacryloxypropyl)tetrakis(trimethylsiloxy)disiloxan, 3-Trimethylsilylpropargylmethacrylat, 3-(Trimethoxysilyl)propylacrylat,
- Vinylmomomeren, die Silikon- oder Silanfunktionen enthalten können; zum Beispiel Vinyl(chlormethyl)dimethoxysilan, Vinylethoxysiloxanpropylethoxysiloxan, Vinyltris(1-methoxy-2-propoxy)silan, Vinyltris(2-methoxyethoxy)silan, Vinyltrimethoxysilan, Vinyltriisopropoxysilan, Vinyltriethoxysilan, Vinyltrichlorsilan, Vinyltri-t-butoxysilan, o-(Vinyloxybutyl)-n-triethoxysilylpropylcarbamat, Vinylmethyldimethoxysilan, Vinylmethyldiethoxysilan, Vinyldimethylethoxysilan, 3-[Tris(trimethylsiloxy)silyl]propylvinylcarbamat, Triethoxysilylpropylmaleinsäure oder 1,3,5-Trivinyl-1,1 ,3,5,5-pentamethyltrisiloxan,
- fluorierten Acrylsäure- und Methacrylsäuremonomeren, wie Dihydroperfluoroctylacrylat, 1,1-Dihydroperfluorbutylacrylat, Trihydroperfluoralkylacrylat, Tetrahydroperfluoralkylacrylat, Tris(trimethylsilyloxy)propylmethacrylat, Perfluorhexylethylthiocarbonylaminoethylmethacrylat, Trifluorethylmethacrylat, Hexafluorisopropylmethacrylat, Hexafluorbutylmethacrylat, Trifluorethylmethacrylat,
- Organosilan-Präpolymerverbindungen, wie α,ω-Bismethacryloxypropylpolydimethylsiloxan, Polydimethylsiloxane mit endständigem/endständigen Vinyl(en), Diphenylsiloxan-Dimethylsiloxan-Copolymere mit endständigen Vinylen, Trifluorpropylmethylsiloxan-Copolymere mit endständigem/endständigen Vinyl(en), Diethylsiloxan-Dimethylsiloxan-Copolymere mit endständigem/endständigen Vinyl(en), Ethylsiloxan-Dimethylsiloxan-Copolymere mit endständigem/endständigen Vinyl(en), Ethylen-Siloxan-Copolymere mit endständigem/endständigen Vinyl(en), Tris(polydimethylsiloxy)silylpropylmethacrylat, Polysiloxanylalkyl(meth)acrylate, Methacryloxypropyl mit endständigem Polydimethylsiloxan, 1,3-Divinyl-1,1,3,3-tetramethyldisiloxan, 2-(Divinylmethylsilyl)ethyltriethoxysilan oder 1,3-Divinyl-1,3-diphenyl-1,3-dimethyldisilan,
- gemischten fluorierten Silan- oder chlorierten Silanverbindungen, wie Fluormethacryloxypropyltris(trimethylsiloxy)silan, 3-Acryloxypropylmethyldichlorsilan, 3-Acryloxypropyltrichlorsilan, 3-Methacryloxypropyltrichlorsilan,
- Silan-/Isocyanatverbindungen wie 3-Isocyanatopropyltriethoxysilan, und
- Vinylmonomeren, wie N-Vinyllactame; zum Beispiel N-Vinylpyrrolidon (NVP)), N-Vinyl-N-methylacetamid (VMA), N-Vinyl-N-ethylacetamid, N-Vinyl-N-ethylformamid, N-Vinylformamid, N-2-Hydroxyethylvinylcarbamat, N-Carboxyalanin-N-vinylester, vorzugsweise CH₃C(O)N(CH₃)-CH=CH₂ (N-Vinyl-N-methylacetamid (VMA)).

8. Zusammensetzung nach einem der Ansprüche 1 bis 7, wobei der Initiator mindestens ein UV-, thermischer und/oder Redoxinitiator ist, ausgewählt aus:
- einem photochemischen Initiator, ausgewählt aus der Gruppe umfassend 2-Hydroxy-4-acryloyloxyethoxybenzophenon, 2-Hydroxy-4-(2-hydroxy-3-methacrylyloxy)propoxybenzophenon; Benzophenon, Acetophenon, Acetonaphthoguinon, Methylethylketon, Valerophenon, Hexanophenon, C-Phenylbutyrophenon, p-Morpholinopropiophenon, Dibenzosuberon, 4-Morpholinobenzophenon, 4-Morpholinodesoxybenzoin, p-Diacetylbenzol, 4-Aminobenzophenon, para-Methoxyacetophenon, 3-Methylanthrachinon, tert-Butylanthrachinon, Anthrachinonester, Benzaldehyd, 9-Acetylphenanthren, 2-Acetylphenanthren, 10-Thioxanthenon, 3-Acetylphenanthren, 3-Acetylindol, 9-Fluorenon, 1-Indanon, 1,3,4-Triacetylbenzol, Thioxanthen-9-on, Xanthen-9-on, 2,4-Dimethylthioxanthon, 2,4-Diethylthioxanthon, 2,4-Diisopropylthioxanthon, 2,4-Dichlorthioxanthon, Benzoin, Benzoinisobutylether, Benzointetrahydropyranylether, Benzoinmethylether, Benzoinethylether, Benzoinbutylether, Benzoinisopropylether, Chlorxanthenon, 7-H-Benzoinmethylether, Benzodeanthracen-7-on, 1-Naphthaldehyd, 4,4'-Bis(dimethylamino)benzophenon, 4-Phenylbenzophenon, 4-Chlorbenzophenon, 4,4'-Bis(dimethylamino)benzophenon, 1-Acetonaphthon, 2-Acetonaphthon, 1-Benzoylcyclohexan-1-ol, 2-Hydroxy-2,2-dimethylacetophenon, 2'2-Dimethoxy-2-phenylacetophenon, 2,2-Diethoxy-2-phenylacetophenon, 1,1-Dichloracetophenon, 1-Hydroxyacetophenon, Acetophenondimethylacetal, o-Methoxybenzophenon, Triphenylphosphin, Tris-o-tolylphosphin, Benzaanthracen-7,12-dion, 2,2-Diethoxyacetophenon, Benzylacetale wie Benzyldimethylacetal, 2-Methyl-1-4-(methylthio)phenyl-2-morpholinopropan-1-on, Anthrachinone wie 2-Methylanthrachinon, 2-Ethylanthrachinon, 2-tert-Butylanthrachinon, 1-Chloranthrachinon, 2-Amylanthrachinon, 2,3-Butandion,
- einem Photoinitiator, ausgewählt aus der Gruppe umfassend Phenylglyoxalsäureester, und deren Kombinationen untereinander oder mit Photopolymerisationspromotoren wie Benzoesäure oder Amine,
- einem thermischen Initiator, ausgewählt aus der Gruppe umfassend 2,2-Azobis(2,4-dimethylpentannitril), 2,2'-Azobisisobutyronitril, 1,1'-Azobis(cyclohexancarbonitril), 2,2'-Azobis(2-methylpropionitril), Kaliumperoxodisulfat, Dibenzoylperoxid, Cyclohexanonperoxid, Di-tert-butylperoxid, Acetylcyclohexylsulfonylperoxid, Diisopropylpercarbonat, tert-Butylperoctoat oder -benzpinakol, Di-t-butylperoxid, Cumolhydroperoxid, Dicumylperoxid, t-Butylperbenzoat und hydroxyliertes N-Oxydamin, wie 2,2,6,6-Tetramethylpiperidin-N-oxyl und 4-Hydroxy-2,2,6,6-tetramethylpiperidin-N-oxyl,
- einem Redox-Initiatorsystem, ausgewählt aus der Gruppe umfassend Oxidationsmittel wie AIBN, Peroxide wie Wasserstoffperoxid, Benzoylperoxid, t-Butyl-hydroperoxid, p-Mentanhydroperoxid, Ammoniumpersulfat, Natriumpersulfat, Kaliumpersulfat, Reduktionsmittel wie Ascorbinsäure, Natriumbisulfit, N,N,N',N'-Tetramethylethylendiamin Natriumformaldehydsulfoxylat, Metallsalze Fe²⁺, Cr²⁺, Zn²⁺, V²⁺, Ti³⁺, Co²⁺, Cu⁺ wie Eisensulfat und N,N-Dialkylaniline wie N,N-Dimethyl-p-toluidin.

9. Hydrogel, das erhalten werden kann durch:
- Hydratation/Quellung, ausgehend von Wasser oder einer wässrigen Lösung, einer vernetzten Zusammensetzung nach einem der Ansprüche 2 bis 8 oder einem der Ansprüche oder
- Polymerisation der vernetzbaren Zusammensetzung nach Anspruch 1 oder einem der Ansprüche 3 bis 8 in Gegenwart eines wassermischbaren protischen oder aprotischen Lösemittels, danach Austausch des Lösemittels mit einem Überschuss Wasser oder einer wässrigen Lösung, oder
- durch Polymerisation der vernetzbaren Zusammensetzung nach Anspruch 1 oder einem der Ansprüche 3 bis 8, in Gegenwart von Wasser oder einer wässrigen Lösung.

10. Herstellungsverfahren einer vernetzten Zusammensetzung wie in Anspruch 2 definiert, bestehend aus dem Umsetzen, in Gegenwart oder Abwesenheit von aprotischem Lösemittel, vorzugsweise in Abwesenheit von Lösemittel, von:
A) mindestens einem polymerisierbaren Monomer oder einer Mischung von polymerisierbaren Monomeren oder einer Mischung von polymerisierbaren Monomeren und Makromonomeren, die mindestens eine polymerisierbare C=C-Funktion vom Acrylat-, Methacrylat-, Maleat-, Fumarat-, Itaconat-, Citraconat-, Styrol- oder Vinyltyp tragen
B) mindestens einem Polyol, ausgewählt aus Oligoglycerolen und linearen, verzweigten und hyperverzweigten Polyglycerolen oder einer Mischung von hyperverzweigtem Polyglycerol und einem Polyol, ausgewählt aus Oligoglycerolen und linearen, verzweigten und hyperverzweigten Polyglycerolen, die im Durchschnitt mehr als 1 Hydroxylgruppen umfassen, die mit einer Gruppe funktionalisiert sind, die eine polymerisierbare C=C-Funktion vom Isocyanatoalkyl(meth)acrylat-, Maleat-, Fumarat-, Itaconat-, Citraconat, Styrol- oder Vinyltyp trägt;
C) einen Radikalinitiator oder eine Mischung von Radikalinitiatoren, die die Polymerisation auf thermischen, Redox- oder photochemischen Wegen initiieren; und
D) gegebenenfalls mindestens einem Zusatzstoff, ausgewählt aus Antioxidationsmitteln, Mitteln zum Anpassen der physiko-chemischen Eigenschaften wie den Elastizitätsmodul, die Zug- und Bruchspannung, Promotoren der Sauerstoffdurchlässigkeit, Weichmachern, Feuchthaltemitteln, Schmiermitteln, Viskositätsmodifikationsmitteln, Kompatibilisierungsmitteln, Färbemitteln, Filtriermitteln, antimikrobiellen Mitteln, therapeutischen Mitteln und Mitteln gegen bakterielle Biofilme.

11. Verfahren nach Anspruch 10, wobei das funktionalisierte hyperverzweigte Polyglycerol in einer Menge von 0,1 bis 99,99 Masse-% des Gesamtgewichts der Bestandteile A) bis D) vorliegt oder wobei der Initiator in einer Menge von 0,02 bis 5 Masse-% des Gesamtgewichts der Bestandteile A) bis D) vorliegt.

12. Verfahren nach einem der Ansprüche 10 oder 11, ferner umfassend einen Formungsschritt der vernetzten Zusammensetzung.

13. Artikel, der durch ein Verfahren nach einem der Ansprüche 10 bis 12 erhalten werden kann.

14. Artikel nach Anspruch 13, der ausgewählt ist aus einer Kontaktlinse oder intraokularen Linse, einem Pflaster, einem Implantat oder einem Wundverband.

15. Verwendung einer Zusammensetzung oder eines Hydrogels nach einem der Ansprüche 1 bis 9 zur Anfertigung einer medizinischen Vorrichtung oder als Vektor einer Zusammensetzung von Interesse, ausgewählt aus therapeutischen Wirkstoffen, Vitaminen, Nährstoffen, Dekontaminationsmitteln oder Schmierprodukten, oder in biomedizinischen, kosmetischen oder sanitären Anwendungen.

## Claims

1. A crosslinkable composition comprising:
A) at least one polymerizable monomer or a mixture of polymerizable monomers or a mixture of monomers and polymerizable macromonomers bearing at least one polymerizable C=C function of the acrylate, methacrylate, maleate, fumarate, itaconate, citraconate, styrenic or vinylic type;
B) at least one polyol selected from oligoglycerols, linear, branched and hyperbranched polyglycerols, or a mixture of hyperbranched polyglycerol and of a polyol selected from the oligoglycerols, and the linear, branched and hyperbranched polyglycerols, comprising on average more than 1 hydroxyl group functionalized with a group bearing a polymerizable C=C function of the isocyanatoalkyl(meth)acrylate, maleate, fumarate, itaconate, citraconate, styrenic or vinylic type;
C) a radical initiator or a mixture of radical initiators that are able to initiate polymerization by thermal, redox or photochemical routes; and
D) optionally at least one additive selected from antioxidants, agents for adjusting the physicochemical properties, agents for promoting, modulating and/or controlling permeability to oxygen, plasticizers, moistening agents, lubricants, viscosity modifiers, compatibilizers, colorants, filtering agents, antimicrobial agents, therapeutic agents and agents against bacterial biofilms.

2. A crosslinked composition able to form a hydrogel polymer, resulting from the crosslinking of:
A) at least one polymerizable monomer or mixture of polymerizable monomers or a mixture of monomers and polymerizable macromonomers bearing at least one polymerizable C=C function of the acrylate, methacrylate, maleate, fumarate, itaconate, citraconate, styrenic or vinylic type;
B) at least one polyol selected from oligoglycerols, linear, branched and hyperbranched polyglycerols or a mixture of hyperbranched polyglycerol and of a polyol selected from oligoglycerols and linear, branched and hyperbranched polyglycerols, comprising on average more than 1 hydroxyl group functionalized with a group bearing a polymerizable C=C function of the isocyanatoalkyl(meth)acrylate, maleate, fumarate, itaconate, citraconate, styrenic or vinylic type;
C) a radical initiator or a mixture of radical initiators capable of initiating polymerization by thermal, redox or photochemical routes; and
D) optionally at least one additive selected from antioxidants, agents for adjusting the physicochemical properties such as Young's modulus, elongation at break and breaking stress, agents for promoting, modulating and/or controlling permeability to oxygen, plasticizers, moistening agents, lubricants, viscosity lowering agents, compatibilizers, colorants, filtering agents, antimicrobial agents, therapeutic agents and agents against bacterial biofilms.

3. The composition as claimed in either one of claims 1 or 2, in which the functionalized hyperbranched polyglycerol is obtained from a hyperbranched polyglycerol having a number average molecular weight between 546 and 100 000 g/mol, and has a degree of branching (DB) between 0.3 and 1.0.

4. The composition as claimed in either one of claims 1 or 2, in which the hyperbranched polyglycerol is a polyglycerol dendrimer or a hyperbranched polyglycerol with a degree of branching between 0.3 and 1.0, or a mixture of hyperbranched polyglycerol and of oligoglycerols or of linear polyglycerols.

5. The composition as claimed in any one of claims 1 to 4, in which the polymerizable C=C function on the functionalized hyperbranched polyglycerol is selected from the isocyanatoalkyl(meth)acrylate, maleic, fumaric, itaconic, citraconic or vinylic groups of structure: 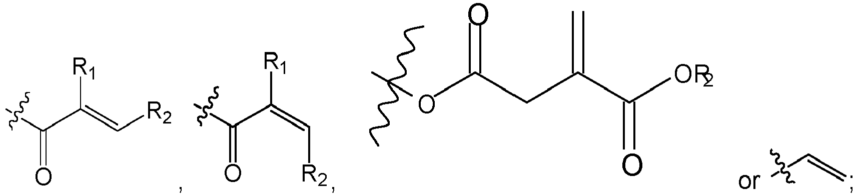 in which R₁ represents H or methyl; R₂ represents H, linear or branched C₁₋₆alkyl, or - C(=O)OR_{2A} in which R_{2A} represents H or linear or branched C₁₋₆alkyl optionally substituted with one or more hydroxyl groups, and R₃ represents H, linear or branched C1-20 alkyl.

6. The composition as claimed in claim 1 to 5, in which the isocyanatoalkyl(meth)acrylate is selected from 2-isocyanatoethylmethacrylate and 2-isocyanatoethylacrylate or a mixture thereof.

7. The composition as claimed in any one of claims 1 to 6, in which constituent A) is selected from:
- acrylic acid or methacrylic acid as well as alkyl (meth)acrylates and derivatives thereof, such as 2-hydroxyethylmethacrylate (HEMA), 2-hydroxyethylacrylate (HEA), methylmethacrylate (MMA), methacrylamide, N,N-dimethyl-diacetoneacrylamide, 2-phosphatoethylmethacrylate, mono-, di-, tri-, tetra-, penta-polyethylene glycol acrylates or methacrylates, N-(3-methacrylamidopropyl)-N,N-dimethylamine, N-(3-methacrylamidopropyl)-N,N,N-trimethylamine, N-(3-acrylamido-3-methylbutyl)-N,N-dimethylamine, N-methacrylamide, 3-hydroxypropyl methacrylate, propyl methacrylate, propyl acrylate, butyl methacrylate, butyl acrylate, pentyl acrylate, pentyl methacrylate, N-(1,1-dimethyl-3-oxobutyl)acrylamide, 2-ethyl-2-(hydroxy-methyl)-1,3-propanediol trimethacrylate, butyl(meth)acrylate, 2-hydroxybutyl methacrylate, 3-hydroxy-2-naphthyl methacrylate, N-(formylmethyl)acrylamide, 2-ethoxyethyl methacrylate, 4-t-butyl-2-hydroxycyclohexyl methacrylate, or the isocyanatoalkyl(meth)acrylates such as 2-isocyanatoethylmethacrylate, 2-isocyanatoethyl acrylate, 4-[(a)-phenyl diazenyl]phenyl-2-methacrylate (4-[(E)-phenyldiazenyl]phenyl-2-methacrylate) or 4-[(a)-phenyl diazenyl]phenyl-2-methacrylate (4-[(E)-phenyldiazenyl]phenyl-2-methacrylate),
- the maleate and fumarate monomers, such as maleic acid, maleic anhydride, dimethyl maleate, diethyl maleate, or the higher maleate esters in which the alkyl chain contains 3 to 20 carbon atoms as well as fumaric acid, dimethyl fumarate, diethyl fumarate or the higher fumarate esters in which the alkyl chain contains 3 to 20 carbon atoms,
- the itaconate monomers; such as itaconic acid, itaconic anhydride, dimethyl itaconate, or the higher itaconate esters in which the alkyl chain contains 3 to 20 carbon atoms,
- the citraconate monomers; such as citraconic acid, citraconic anhydride, dimethyl citraconate, or the higher citraconate esters in which the alkyl chain contains 3 to 20 carbon atoms,
- the (meth)acrylic monomers that may contain silicone or silane functions; for example tris(trimethylsiloxy)silylpropyl methacrylate, bis(trimethylsiloxy)methylsilylpropyl methacrylate, pentamethyldisiloxanepropyl methacrylate, tris(trimethylsiloxy) silylpropyloxyethyl methacrylate, tris(trimethylsiloxy)-silylpropyl methylacryloxyethylcarbamate, tris(trimethylsiloxy)silylpropylglycerol methacrylate, phenyltetramethyl-disiloxanylethyl acrylate, 3-methacryloxypropylbis(trimethylsiloxy)methylsilane, methyl-di(trimethylsiloxy)methacryloxymethyl silane, (3-methacryloxy-2-hydroxypropyloxy) propyl bis(trimethylsiloxy)methylsilane, 3-acryloxypropyl tris(trimethylsiloxy)silane, 3-acryloxypropyltrichlorosilane, 3-methacryloxypropyl dimethyl ethoxysilane, 3-methacryloxypropyl methyl diethoxysilane, methacryloxypropylbis(trimethylsiloxy)methylsilane, tristrimethylsilyloxysilylpropyl methacrylate, bis(methacryloxypropyl) tetramethyldisiloxane, acryloxymethyltrimethylsilane, p-(t-butyldimethylsiloxy)styrene, 1,3-bis(3-methacryloxypropyl)tetrakis(trimethylsiloxy)disiloxane, 3 trimethylsilylpropargylmethacrylate, 3-(trimethoxysilyl)propyl acrylate,
- vinyl monomers, which may contain silicone or silane functions; for example vinyl(chloromethyl)dimethoxysilane, vinylethoxysiloxane-propylethoxysiloxane, vinyltris(1-methoxy-2-propoxy)silane, vinyltris(2-methoxyethoxy)silane, vinyltrimethoxysilane, vinyltriisopropoxysilane, vinyltriethoxysilane, vinyltrichlorosilane, vinyl tri-t-butoxysilane, o-(vinyloxybutyl)-n-triethoxysilylpropyl carbamate, vinylmethyldimethoxysilane, vinylmethyldiethoxysilane, vinyldimethylethoxysilane, 3-[tris(trimethylsiloxy)silyl] propylvinyl carbamate, triethoxysilylpropyl maleic acid or 1,3,5-trivinyl-1,1,3,5,5-pentamethyltrisiloxane,
- fluorinated acrylic and methacrylic monomers, such as dihydroperfluorooctyl acrylate, 1,1-dihydroperfluorobutyl acrylate, trihydroperfluoroalkyl acrylate, tetrahydroperfluoroalkylacrylate, tris(trimethylsilyloxy)propyl methacrylate, perfluoro hexylethylthiocarbonylaminoethyl methacrylate, trifluoroethyl methacrylate, hexafluoroisopropyl methacrylate, hexafluorobutyl methacrylate, trifluoroethyl methacrylate,
- organosilane prepolymer compounds, such as α,ω-bismethacryloxy-propyl polydimethylsiloxane, polydimethylsiloxanes with vinylic termination(s), diphenylsiloxane-dimethylsiloxane copolymers with a vinylic termination, trifluoropropylmethylsiloxane copolymers with vinylic termination(s), diethylsiloxane-dimethylsiloxane copolymers with vinylic termination(s), ethylsiloxane-dimethylsiloxane copolymers with vinylic termination(s), ethylene-siloxane copolymers with vinylic termination(s), tris(polydimethylsiloxy)silylpropyl methacrylate, polysiloxanylalkyl (meth)acrylates, methacryloxypropyl with polydimethylsiloxane termination, 1,3-divinyl-1,1,3,3-tetramethyldisiloxane, 2-(divinylmethylsilyl)ethyltriethoxysilane, or 1,3-divinyl-1,3-diphenyl-1,3-dimethyldisilane,
- fluorinated / silane or chlorinated / silane mixed compounds, such as fluoro-methacryloxypropyl tris(trimethylsiloxy) silane, 3-acryloxy propyl methyl dichlorosilane, 3-acryloxypropyltrichlorosilane, 3-methacryloxypropyl trichlorosilane,
- the silane / isocyanate compounds such as 3-isocyanatopropyltriethoxysilane, and
- vinyl monomers, such as N-vinyl lactams; for example N-vinyl pyrrolidone (NVP)), N-vinyl-N-methyl acetamide (VMA), N-vinyl-N-ethyl acetamide, N-vinyl-N-ethyl formamide, N-vinyl formamide, N-2-hydroxyethyl vinyl carbamate, N-carboxy-alanine N-vinyl ester, preferably CH₃C(O)N(CH₃)-CH=CH₂ (N-vinyl-N-methyl acetamide (VMA)).

8. The composition as claimed in any one of claims 1 to 7, in which the initiator is at least one UV, thermal and/or redox initiator, selected from:
- a photochemical initiator selected from the group comprising 2-hydroxy-4-acryloyloxyethoxy benzophenone, 2-hydroxy-4-(2-hydroxy-3-methacrylyloxy) propoxybenzophenone; benzophenone, acetophenone, acetonaphthoquinone, methyl ethyl ketone, valerophenone, hexanophenone, C-phenylbutyrophenone, p-morpholinopropiophenone, dibenzosuberone, 4-morpholinobenzophenone, 4-morpholinodeoxybenzoin, p-diacetylbenzene, 4-aminobenzophenone, para-methoxyacetophenone, 3-methylanthraquinone, tert-butylanthraquinone, the esters of anthraquinone, benzaldehyde, 9-acetylphenanthrene, 2-acetylphenanthrene, 10-thioxanthenone, 3-acetylphenanthrene, 3-acetylindole, 9-fluorenone, 1-indanone, 1,3,4-triacetylbenzene, thioxanthen-9-one, xanthen-9-one, 2,4-dimethylthioxanthone, 2,4-diethylthioxanthone, 2,4-diisopropylthioxanthone, 2,4-dichlorothioxanthone, benzoin, benzoin isobutyl ether, benzoin tetrahydropyranyl ether, benzoin methyl ether, benzoin ethyl ether, benzoin butyl ether, benzoin isopropyl ether, chloroxanthenone, 7-H-benzoin methyl ether, benzodeanthracen-7-one, 1-naphthaldehyde, 4,4'-bis(dimethylamino)benzophenone, 4-phenylbenzophenone, 4-chlorobenzophenone, 4,4'-bis(dimethylamino)benzophenone, 1-acetonaphthone, 2-acetonaphthone, 1-benzoylcyclohexan-1-ol, 2-hydroxy-2,2-dimethylacetophenone, 2'2-dimethoxy-2-phenylacetophenone, 2,2-diethoxy-2-phenylacetophenone, 1,1-dichloroacetophenone, 1-hydroxyacetophenone, acetophenone dimethyl acetal, o-methoxybenzophenone, triphenylphosphine, tris-o-tolylphosphine, benzaanthracene-7,12-dione, 2,2-diethoxyacetophenone, the benzyl acetals such as benzyl dimethyl acetal, 2-methyl-1-4-(methylthio)phenyl-2-morpholinopropan-1-one, the anthraquinones such as 2-methylanthraquinone, 2-ethylanthraquinone, 2-tert-butylanthraquinone, 1- chloroanthraquinone, 2-amylanthraquinone, 2,3-butanedione,
- a photoinitiator selected from the group comprising esters of phenylglyoxalic acid, and combinations thereof with one another or with promoters of photopolymerization such as benzoic acid or amines,
- a thermal initiator selected from the group comprising 2,2-azobis(2,4-dimethylpentanenitrile), 2,2'-azobisisobutyronitrile, 1,1'-azobis(cyclohexanecarbonitrile), 2,2'-azobis(2-methylpropionitrile), potassium peroxodisulfate, dibenzoyl peroxide, cyclohexanone peroxide, di-tert-butyl peroxide, acetyl cyclohexylsulfonyl peroxide, diisopropyl percarbonate, tert-butyl peroctoate or benzpinacol, di-t-butyl peroxide, cumene hydroperoxide, dicumyl peroxide, t-butyl perbenzoate, and hydroxylated N-oxide amine, such as 2,2,6,6-tetramethylpiperidine-N-oxyl and 4-hydroxy-2,2,6,6-tetramethylpiperidine-N-oxyl,
- a redox initiator system selected from the group comprising oxidants such as AIBN, the peroxides such as hydrogen peroxide, benzoyl peroxide, t-butylhydroperoxide, p-mentane hydroperoxide, ammonium persulfate, sodium persulfate, potassium persulfate, reducing agents such as ascorbic acid, sodium bisulfite, N,N,N',N'-tetramethylethylene diamine formaldehyde sodium sulfoxylate, the Fe²⁺, Cr²⁺, Zn²⁺, V²⁺, Ti³⁺, CO₂⁺, Cu⁺ metal salts such as iron sulfate, and the N,N-dialkylanilines such as N,N-dimethyl-p-toluidine.

9. A hydrogel obtainable by:
- hydration/swelling, from water or an aqueous solution, of a crosslinked composition as claimed in any one of claims 2 to 8 or any one of the claims, or,
- polymerization of the crosslinkable composition as claimed in claim 1 or any one of the claims 3 to 8, in the presence of a water-miscible protic or aprotic solvent, and then exchange of the solvent with an excess of water or an aqueous solution, or
- by polymerization of the crosslinkable composition as claimed in claim 1 or any one of the claims 3 to 8, in the presence of water or an aqueous solution.

10. A method for preparing a crosslinked composition as defined in claim 2 consisting of reacting, in the presence or absence of aprotic solvent, preferably in the absence of solvent:
A) at least one polymerizable monomer or mixture of polymerizable monomers or a mixture of monomers and polymerizable macromonomers bearing at least one polymerizable C=C function of the acrylate, methacrylate, maleate, fumarate, itaconate, citraconate, styrenic or vinylic type
B) at least one polyol selected from oligoglycerols and linear, branched and hyperbranched polyglycerols, or a mixture of hyperbranched polyglycerol and of a polyol selected from oligoglycerols and linear, branched and hyperbranched polyglycerols, comprising on average more than 1 hydroxyl groups functionalized with a group bearing a polymerizable C=C function of the isocyanatoalkyl(meth)acrylate, maleate, fumarate, itaconate, citraconate, styrenic or vinylic type;
C) a radical initiator or a mixture of radical initiators that are able to initiate polymerization by thermal, redox or photochemical routes; and
D) optionally at least one additive selected from antioxidants, agents for adjusting the physicochemical properties such as Young's modulus, elongation at break and breaking stress, promoters of permeability to oxygen, plasticizers, moistening agents, lubricants, viscosity modifiers, compatibilizers, colorants, filtering agents, antimicrobial agents, therapeutic agents and agents against bacterial biofilms.

11. The method as claimed in claim 10, in which the functionalized hyperbranched polyglycerol is present at a rate from 0.1 to 99.99% by weight of the total weight of constituents A) to D), or in which the initiator is present at a rate from 0.02 to 5% by weight of the total weight of constituents A) to D).

12. The method as claimed in either one of claims 10 or 11, further comprising a step of molding the crosslinked composition.

13. An article obtainable by the method as claimed in any one of claims 10 to 12.

14. The article as claimed in claim 13, which is selected from a contact lens or intraocular lens, a patch, an implant or a dressing.

15. The use of a composition or of a hydrogel as claimed in any one of claims 1 to 9 for manufacturing a medical device, or as a vector of a compound of interest selected from therapeutic active ingredients, vitamins, nutrients, decontaminating agents or lubricants, or in biomedical, cosmetic, or health-related applications.
